# EUROPEAN PATENT APPLICATION

(11) **EP 4 834 352 A1**
(43) Date of publication of application: **04.11.2026**
(21) Application number: 25172931.5
(22) Date of filing: 28.04.2025
(51) Int. Cl.: A61K 31/7088, A61K 31/711, A61K 39/395, A61P 35/02

(54) **COMPOSITION COMPRISING AN IL-7 ANTAGONIST AND A TLR AGONIST**

(71) Applicant: AVA Lifescience GmbH, 79189 Bad Krozingen (DE)
(72) Inventor: Jumaa-Weinacht, Hassan, Bad Krozingen (DE); Setz, Corinna, Bad Krozingen (DE); El Ayoubi, Oma, Bad Krozingen (DE)
(74) Representative: Vossius & Partner Patentanwälte Rechtsanwälte mbB

(57) **Abstract**

The invention relates to a composition or a kit comprising a Toll-like receptor agonist and an Interleukin-7 (IL-7) antagonist as well as methods and uses for treating or preventing lymphoblastic leukemia.

## Description

The present invention relates to the field of IL-7 antagonist compositions. In particular, the invention relates to a composition or a kit comprising a Toll-like receptor agonist and an Interleukin-7 (IL-7) antagonist as well as methods and uses for treating or preventing lymphoblastic leukemia.

### RELATED ART

B cell development is a complex process that is tightly controlled by a network of soluble mediators, multiple interacting signaling cascades and a diverse set of transcription factors (Lee et al., 1989; Ishihara et al., 1991; Rolink et al., 1995). Productive Ig gene recombination enables expression of B cell antigen receptors (BCRs), which are essential for controlling B cell development by mediating the selection of functional and self-tolerant B cells, thereby conferring immune protection and avoiding autoimmunity (Shlomchik, 2008; Pelanda and Torres, 2012). Thus, defects in signal transduction in developing B cells can impair normal development and lead to uncontrolled proliferation and leukemia, or immunodeficiency or autoimmunity.

Acute lymphoblastic leukemia (ALL) is a heterogeneous disease with multiple, prognostically relevant genetic aberrations and characterized by the over-production of immature white blood cells (Inaba et al., 2013). In adults, 20-30% of patients with an aggressive form of ALL, Ph+ ALL, express the BCR-ABL oncogene as the result of the Philadelphia-translocation t(9;22)(q34;q11) and have a very poor prognosis (Lee et al., 2011). The oncoprotein BCR-ABL is a constitutively active tyrosine kinase, which promotes uncontrolled cell proliferation (Deininger et al., 2000).

Due to the constitutive kinase activity of BCR-ABL, addition of tyrosine kinase inhibitors (TKIs) such as imatinib mesylate to intensive chemotherapy has improved the outcome of BCR-ABL1-positive leukemia to a 5-year disease-free survival rate in children (70 ± 12%, n =28) (Schultz et al., 2014, Leukemia 28, 1467-1471). Nevertheless, Ph+ ALL patients still suffer from poor prognosis in both children and adults as relapse frequently occurs after stem cell transplantation.

Interleukin-7 receptor (IL7R) has been shown to interact with the chemokine receptor CXCR4 to recruit BCR-ABL1 and JAK kinases in close proximity. Treatment with BCR-ABL1 kinase inhibitors results in elevated expression of IL7R which enables the survival of transformed cells when IL7 was added together with the kinase inhibitors. Anti-IL7R antibodies were used in an approach for preventing leukemia development in xenotransplantation models using patient-derived Ph+ ALL cells (Abdelrasoul et al., 2020, Nature Communications 11:3194, DOI:1038/s41467-020-16927-w).

In another study, it was shown that combined treatment using dasatinib and ruxolitinib, the latter is an inhibitor of IL7R signaling and of kinases JAK1/JAK2, reduced leukemia engraftment and prolonged survival. However, these mice eventually relapsed and died from leukemia which suggest that in vivo treatment with ruxolitinib is inefficient.

Thus, there is still a strong need for an efficient treatment of patients suffering from lymphoblastic leukemia including Ph+ ALL.

### SUMMARY OF THE INVENTION

The inventors showed that the TLR signaling machinery is already functional in early B cells. For example, both Ig-k- and Ig-k+ proliferated less in presence of CpG as compared with unstimulated cells. This finding supports the conclusion that CpG activates the differentiation program of BCPs while suppressing proliferation. Activating TLR9 may thereby contribute to B cell differentiation and maturation and may play a role in overcoming differentiation blockades.

Combination of CpG with other TLR ligands for in vitro stimulation of BCR-ABL-transformed BCPs and measurement of survival showed that stimulation with different TLR ligands or combinations thereof can reproduce comparable effects as treatment with Imatinib alone. For example, combination of CpG with the TLR4 ligand lipopolysaccharide (LPS) induces a synergistic effect.

Further, the inventors showed that BCR-ABL1-transformed B cells, which are blocked in differentiation and do not express BCR, show strong activation upon CpG-exposure. These data indicate that BCR-ABL1-expression confers sensitivity to TLR9 stimulation. While stimulating BCR-ABL1-transformed B cells with CpG, a considerable number of cells was lost which indicates that CpG treatment leads to cell death in BCR-ABL1-transformed B cells. This is also confirmed in BCR-ABL1-transformed BCP-ALL cells of human origin and in an entire organism. Moreover, a combination of a tyrosine kinase inhibitor, such as Imatinib, is highly efficient in limiting survival of BCR-ABL1-transformed B cells, demonstrating that the combination of both substances elicits synergistic effects. Taken together, the inventor's findings suggest that CpG-ODNs by themselves or in combination with a tyrosine kinase inhibitor represents a powerful novel approach in the treatment and/or prevention of Ph+ ALL.

In addition, the inventors demonstrated that the combined treatment of CpG and an anti-IL7R antibody resulted in a clear reduction of the leukemic cells in the blood of recipient mice suggesting that this combined treatment might be an effective treatment of leukemia.

Thus, the present invention relates to, inter alia, the following items:
1. A composition comprising a Toll-like receptor agonist and an Interleukin-7 (IL-7) antagonist.
2. A kit comprising a Toll-like receptor agonist and an Interleukin-7 (IL-7) antagonist.
3. The composition of embodiment 1 or kit of embodiment 2, wherein the Toll-like receptor is selected from the group consisting of TLR2, TLR4, TLR5, TLR6, TLR7, and TLR9; wherein preferably the Toll-like receptor is selected from the group consisting of TLR2, TLR6, TLR7 and TLR9; wherein more preferably the Toll-like receptor is TLR9.
4. The composition or kit of anyone of the preceding embodiments, wherein the Toll-like receptor agonist is a nucleic acid molecule, preferably a single stranded nucleic acid molecule, more preferably a single-stranded DNA or RNA molecule, again more preferably a single-stranded DNA molecule.
5. The composition or kit of anyone of the preceding embodiments, wherein the Toll-like receptor agonist is a CpG oligonucleotide comprising at least one preferably at least two unmethylated CpG dinucleotides (CpG motifs), preferably the CpG is a DNA molecule containing at least one preferably at least two unmethylated CpG dinucleotides.
6. The composition or kit of anyone of the preceding embodiments, wherein the CpG oligonucleotide comprises or consists of a class A, class B or class C CpG oligonucleotide, preferably a class B CpG oligonucleotide.
7. The composition or kit of anyone of the preceding embodiments, wherein all or at least one of the internucleotide linkages of the CpG oligonucleotide are phosphorothioate linkages.
8. The composition or kit of anyone of the preceding embodiments, wherein the CpG oligonucleotide comprises or consists of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684; or the CpG oligonucleotide comprises or consists of a variant of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1 to 4 nucleotide(s) is/are substituted, added or deleted, and the variant comprises at least one, preferably at least two CpG motifs.
9. The composition or kit of anyone of the preceding embodiments, wherein the IL-7 antagonist is an interleukin-7 receptor (IL7R), binds to an IL7R or binds to IL-7.
10. The composition or kit of anyone of the preceding embodiments, wherein the IL-7 antagonist binding to IL7R binds to an alpha chain of IL7R (CD127).
11. The composition or kit of anyone of the preceding embodiments, wherein the IL-7 antagonist binding to IL7R or IL-7 is an antibody, or an antigen binding fragment thereof.
12. The composition or kit of anyone of the preceding embodiments, wherein the IL-7 antagonist binding to IL7R or IL-7 is a monoclonal antibody, or an antigen binding fragment thereof.
13. The composition or kit of anyone of the preceding embodiments, wherein the IL-7 antagonist is an antibody, preferably a monoclonal antibody, binding to IL7R (IL7R antibody), or an antigen binding fragment thereof, wherein the IL7R antibody, or the antigen binding fragment thereof binds to the IL7R alpha chain.
14. The composition or kit of anyone of the preceding embodiments, further comprising a kinase inhibitor, preferably tyrosine kinase inhibitor, more preferably a Bcr-Abl tyrosine-kinase inhibitor.
15. The composition or kit of anyone of the preceding embodiments, further comprising a pharmaceutically acceptable carrier and/or an anti-cancer drug.
16. The composition or kit of any one of the preceding embodiments
   for use in treating or preventing lymphoblastic or lymphocytic leukemia,
   preferably acute lymphoblastic leukemia or chronic lymphatic leukemia,
   more preferably acute lymphoblastic leukemia (ALL).
17. A method for treating or preventing lymphoblastic or lymphocytic leukemia, said method comprises administering to a subject a therapeutically effective dose of the composition of any one of the preceding embodiments or a therapeutically effective dose of the Toll-like receptor agonist and the Interleukin-7 (IL-7) antagonist of the kit of any one of the preceding embodiments.
18. The composition or kit for use according to embodiment 16, or the method of embodiment 17, wherein the ALL is B-cell precursor ALL.
19. The composition or kit for use according to embodiment 16 or 18, or the method of embodiment 17 or 18, wherein the ALL is Ph-negative, Ph-positive or Ph-like ALL, preferably Ph-positive or Ph-like ALL, more preferably Ph-positive ALL.
20. The composition or kit for use according to embodiment 16 or 18, or the method of embodiment 17 or 18, wherein the ALL is BCR::ABL1-like ALL or characterized by the presence of a fusion gene encoding Bcr-Abl (Bcr-Abl+) or a Philadelphia chromosome (Philadelphia chromosome-positive, Ph+), preferably the ALL is Bcr-Abl+ or Ph+.
21. The composition or kit for use according to any one of embodiment 16, 18 or 19, or the method of any one of embodiment 17, 18 or 19, wherein the patient is resistant to treatment with kinase inhibitors, preferably tyrosine kinase inhibitors, more preferably Bcr-Abl tyrosine-kinase inhibitors.

### DETAILED DESCRIPTION OF THE INVENTION

In a first aspect, the invention relates to a composition comprising a Toll-like receptor agonist and an Interleukin-7 (IL-7) antagonist.

### TLR

Toll-like receptors (TLRs) belong to a family of pattern recognition receptors hat constitute the first line of defense system against microbes. They can recognize both invading pathogens and endogenous danger molecules released from dying cells and damaged tissues and play a key role in linking innate and adaptive immunity. By activating specific TLRs, it is possible to stimulate and control specific types of innate immune responses that can be harnessed to enhance adaptive responses. TLRs are type I transmembrane proteins with 20-27 extracellular leucine-rich repeats (LRR) for the recognition of PAMP/DAMP, transmembrane domains, and intracellular toll-interleukin-1 (IL-1) receptor domains required for the activation of downstream signal transduction pathways (Gao et al., 2017). The extracellular domains of TLR contain glycan moieties that serve as binding sites for ligands (Akira et al., 2006). The main characteristics that distinguish different TLRs are ligand specificity, signal transduction pathways, and subcellular localization (Singh et al., 2014). To date, 10 human and 13 murine subtypes of TLR have been identified, although TLR10 is non-functional in the mouse.

In preferred embodiments, the term TLR refers to human TLRs, i.e., TLR1-10. More preferably, the Toll-like receptor is selected from the group consisting of TLR2, TLR4, TLR5, TLR6, TLR7, and TLR9. Again more preferably, the Toll-like receptor is selected from the group consisting of TLR2, TLR6, TLR7 and TLR9. Most preferably, the Toll-like receptor is TLR9.

TLRs recognize conserved pathogen-associated molecular patterns (PAMPs) expressed on a wide array of microbes, as well as endogenous DAMPs released from stressed or dying cells.

The term "TLR agonist" as used herein preferably refers to a compound that is able to enhance, induce or modulate an immune reaction (i.e., an immunostimulatory agent) by modulating, preferably activating, TLR signaling. The ability of a compound of the invention to act as a TLR agonist may be determined using pharmacological assays well known to the art, including the procedures disclosed by Lee et al. (PNAS, 100(11), pp. 6646-6651, 2003). Preferably, the immunostimulatory agents binds and activates TLR. TLR agonists are disclosed in the art and include for example the following:

| **TLR** | **Cellular localization** | **Adaptor molecule** | **Ligand/agonist** |
|---|---|---|---|
| TLR1-TLR2 | Plasma membrane | MyD88 | Triacylated lipoproteins, lipoteichoic acid, peptidoglycans, Bacterial lipoprotein, Zymosan |
| | | | Pam₃CSK₄ |
| TLR2-TLR6 | Plasma membrane | MyD88 | Diacylated lipopeptides |
| | | | HSPs, HMGB1, uric acid, fibronectin, ECM proteins, Macrophage-activating lipopeptide 2, Pam2CSK₄, Pam₃CSK₄ |
| TLR3 | Endosome | TRIF | dsRNA |
| | | | Poly I:C, poly-ICLC, poly(I:C12,U) poly(A:U) |
| TLR4 | Plasma membrane or endosome | MyD88 or TRIF | LPS, lipoteichoic acid |
| | | | β-defensin 2, fibronectin EDA, HMGB1, snapin, tenascin C, Monophosphoryl lipid-A (MPL) derivative |
| TLR5 | Plasma membrane | MyD88 | Flagellin and recombinants thereof |
| TLR7-TLR8 | Endosome | MyD88 | ssRNA |
| | | | CpG-A, Poly G10, Poly G3, Thiazoquinoline and imidazoquinoline derivatives (e.g., resiquimod, imiquimod); TLR7 agonist vesatolimod (GS-9620)101 and TLR8 agonist GS-9688 |
| TLR9 | Endosome | MyD88 | Unmethylated CpG DNA, DNA: RNA hybrids |
| TLR10 | Surface | MyD88 | Unknown natural ligand |
| | | | Pam₃CSK₄, PamCysPamSK₄ |
| TLR11 | - | - | Flagellin and Profilin |
| TLR12 | - | - | Profilin |
| TLR13 | Endosome | MyD88 | VSV, 23S rRNA-derived oligoribonucleotide |

### TLR9 agonist

Toll-like receptor 9 (TLR9) is expressed on human B cells and plasmacytoid dendritic cells (pDCs), thereby inducing Th1-dominated immune responses. TLR9 (CD289) recognizes unmethylated cytidine-phospho-guanosine (CpG) motifs found in microbial DNA, which can be mimicked using synthetic CpG-containing oligodeoxynucleotides (CpG-ODNs). CpG-ODNs are known to enhance antibody production and to stimulate T helper 1 (Th1) cell responses (Coffman et al., Immunity, 33:492-503, 2010). CpG oligonucleotides are classified as class A (D-type), class B (K-type), class C, class P, and class S. The degree of B cell activation varies between the classes with CpG-A ODNs being weak, CpG-C ODNs being good, and CpG-B ODNs being strong B cell activators. Oligonucleotide TLR9 agonists of the present disclosure are preferably good B cell activators (CpG-C ODN) or more preferably strong (CpG-B ODN) B cell activators.

TLR9 is described in the Online Mendelian Inheritance in Man database (OMIM) under Accession no. *605474, and in the UniGene database under Cluster Hs.87968 (Homo sapiens), Mm.44889 (Mus musculus), and Rn.92495 (Rattus norvegicus). Nucleic acid sequences encoding a TLR9 include those of GenBank accession nos. BC032713.1 (Homo sapiens), NM-031178.1 (Mus musculus) and NM-198131.1 (Rattus norvegicus).

In some embodiments, the TLR or TLR9 comprises a full length TLR or TLR9 protein or a functional fragment thereof, i.e., a fragment which retains the properties of TLR signaling or, in the case of TLR9, being activated by unmethylated CpGs. The TLR9 fragment preferably comprises one or more regions selected from the group consisting of LRR region 1, LRR region 2; transmembrane domain; Toll/IL-1 Resistance (TIR) domain; a ligand binding domain, e.g., a region including a CXXC motif; e.g., CRRC or CMEC; and a localization signal domain. In some embodiments, the TLR or TLR9is a fusion protein, e.g., including a fluorescent protein.

The term "TLR9 agonist" as used herein preferably refers to a CpG comprising compound that is able to enhance, induce or modulate an immune reaction (i.e., an immunostimulatory agent) by modulating, preferably activating, TLR9 signaling. Preferably, the immunostimulatory agents binds and/or activates TLR9. The ability of a compound of the invention to act as a TLR9 agonist may be determined using pharmacological assays well known to the art (Lee et al., PNAS, 100(11), pp. 6646-6651, 2003; iLite TLR9 Assay Ready Cells; TLR9 Human TLR Cell Based Agonist Assay in PBMC by Eurofins). TLR9 agonists according to the invention are disclosed in the art, for example in EP2650369A1 and EP4074334A1.

In preferred embodiments, the TLR agonist, especially the TLR9 agonist, is a nucleic acid molecule, preferably a single stranded nucleic acid molecule, more preferably a single-stranded DNA or RNA molecule, again more preferably a single-stranded DNA molecule.

The TLR9 agonist can be a chimeric protein and/or labeled.

### CpG

In preferred embodiments, the TLR agonist, especially the TLR9 agonist, is a CpG oligonucleotide (herein mentioned synonymously also as CpG or CpG-ODN). CpGs are recognized by TLR9, which leads to immunostimulatory effects. CpG can be synthesized using methods known in the art or obtained from commercial sources (e.g., Apara Bioscience; InvivoGen, San Diego, Calif.; MWG-Biotech, High Point, N.C.; Invitrogen, Carlsbad, Calif.).

The term "oligonucleotide", as used herein, is defined as it is generally understood by the skilled person. Preferably, the term "oligonucleotide", as used herein, refers to as a molecule comprising two or more covalently linked nucleosides or nucleotides. The oligonucleotide can have a length from 2 to 100, or 4 to 80, or 4 to 60, or preferably 4 to 40 nucleotides,

As used herein, the term CpGs refers to a natural or synthetic single-stranded oligonucleotide containing one or more CpG dinucleotides (CpG motifs), wherein C represents cytosine, G represents guanine and p represents a phosphodiester or phosphorothioate bond. CpGs can be microbial CpG, such as bacterial, viral, fungal, and parasite CpG, or preferably synthetic CpGs.

In preferred embodiments, the CpG is a DNA or RNA molecule, preferably a DNA molecule.

In preferred embodiments, the CpG comprises non-methylated CpG motifs. Preferably, the CpG comprising at least one preferably at least two unmethylated CpG dinucleotides (CpG motifs). More preferably the CpG is a DNA molecule containing at least one preferably at least two unmethylated CpG dinucleotides. More preferably, the CpG is non-methylated, all nucleotides are unmethylated.

In preferred embodiments, the CpG does not comprise a TTAGGG motif.

The CpG may contain a modification of the phosphate group. For example, in addition to phosphodiester linkages, phosphate modifications include, but are not limited to, methyl phosphonate, phosphorothioate, phosphoramidate (bridging or non-bridging), phosphotriester and phosphorodithioate, and may be used in any combination. Other non-phosphate linkages may also be used. In some embodiments, the oligonucleotides comprise at least one phosphorothioate linkage. In some embodiments, the CpG comprise at least one phosphodiester linkage. In some embodiments, the CpG comprises a combination of phosphate linkages in the phosphate backbone, such as a combination of phosphodiester and phosphorothioate linkages. The CpG of the present disclosure include at least one, two or three internucleotide phosphorothioate ester linkages. In some embodiments, when a plurality of CpG molecules is present in a pharmaceutical composition comprising at least one excipient, both stereoisomers of the phosphorothioate ester linkage are present in the plurality of CpG molecules. In some embodiment, all of the internucleotide linkages of the CpG are phosphodiester linkages, or said in another way, the CpG has a phosphodiester backbone. In more preferred embodiments, all of the internucleotide linkages of the CpG are phosphorothioate linkages, or said in another way, the CpG has a phosphorothioate backbone.

In preferred embodiments, the CpG comprises at least one modification. Preferably, the modified CpG is still a single-stranded oligonucleotide containing at least one unmethylated CpG motif. Modified CpGs are still able to enhance, induce or modulate an immune reaction by modulating, preferably activating, TLR9 signaling. Preferably, the modified CpGs are still able to bind to and/or activate TLR9.

Preferably, the CpG comprises at least one modified nucleoside. The term "modified nucleoside" preferably is a nucleoside that includes a modified heterocyclic base, a modified sugar moiety, or any combination thereof. In some embodiments, the modified nucleoside is a non-natural pyrimidine or purine nucleoside. For purposes of the invention, a modified nucleoside, a pyrimidine or purine analog or non-naturally occurring pyrimidine or purine can be used interchangeably and refers to a nucleoside that includes a non-naturally occurring base and/or non-naturally occurring sugar moiety. For purposes of the invention, a base is considered to be non-natural if it is not guanine, cytosine, adenine, thymine or uracil.

In preferred embodiments, the CpG comprises chemical modifications selected from the group consisting of methylphosphonate linkages, 2'-alkyl or 3'-deoxy or -alkyl ribonucleosides, non-nucleotide linkers, abasic nucleotides, preferably in the flanking sequences, e.g., at the fourth to sixth nucleotide positions 5' to the CpG dinucleotide.

In a preferred embodiment, the CpG has at least one free 5'-end. In preferred embodiments, the CpG comprises chemical modifications selected from the group consisting of modifications of the 3'OH or 5'OH group, modifications of the nucleotide base, modifications of the sugar component, and modifications of the phosphate group. In some embodiments, the CpG comprises a non-canonical base. In some embodiments, the modified nucleoside is selected from the group consisting of 2'-deoxy-7- deazaguanosine, 2'-deoxy-6-thioguanosine, arabinoguanosine, 2'-deoxy-2'substituted- arabinoguanosine, and 2'-0-substituted-arabinoguanosine.

In some embodiments, a CpG has a length of less than 100, 50, 45, 40, 35, 33, 32, 31, 30, 29, 28, 27, 26, 25 nucleotides in length. In some embodiments, the oligonucleotide has a length of at least 3, 5, 8, 10, 11, 12, 13, 14, 15, 16, 17, 18, 19 nucleotides. In preferred embodiments, the TLR9 agonist is an CpG of from 3-100, 5-50, 10-30, 11-29, 12-28, 13-27, 14-26, 15-25, 16-24 17-23, 18-25 nucleotides in length. In very preferred embodiments, the TLR9 agonist is a CpG from 10 to 35, more preferably 18-25 nucleotides in length.

In very preferred embodiments, the CpG comprises or consists of a class A, class B or class C CpG oligonucleotide, preferably a class B CpG oligonucleotide. Class B CpGs preferably and typically have a linear structure with a phosphorothioate skeleton, and typically do not form a higher-order structure. Class A CpGs preferably and typically have a phosphodiester linkage in the center, and poly-G motifs at both ends that form a higher-order structure called G-tetrad.

In very preferred embodiments, the CpG of the invention comprises or consists of the following CpGs:
Class **A:** D35-CpG, ODN1585, ODN2216, ODN2336, etc.
Class **B:** ODN1018, K3-CpG, CPG T1, T2, T3, ODNBW006, ODN D-SL01, ODN1668, ODN1826, ODN2006, ODN2006, ODN2007, ODN684, CpG T1, CpG T2, CpG T3, CpG D2, etc.
Class **C:** ODN D-SL03, ODN 2395, ODN M362, etc.

Other examples include CpGs include CpG-28, CpG-685 (GNKG-168), CpG-ODN C274, KSK-13 (KSK-CpG), CpG ODN, 10104 (CpG-10104), CpG ODN-1585, ODN-5890, 1018-ISS, EMD-1201081 (HYB-2055, IMO-2055), and the like.

The CpG oligonucleotide used can be a commercially available product or a product obtained according to a known production method.

In very preferred embodiments, the CpG of the invention is an oligodeoxynucleotide which comprises one or more CpG motifs, more preferably two or more CpG motifs, has preferably a length 18-25 nucleotides, and is a fully thio-modified sequences. In more preferred embodiments, the CpG of the invention is an oligodeoxynucleotide which comprises one or more CpG motifs, more preferably two or more CpG motifs, has preferably a length 18-25 nucleotides, is a fully thio-modified sequences, and includes or consists of a CpG of class B.

In other embodiments, the CpG comprises two or more copies of 5'-TCGT-3' motif or 5'-TCGTCGT-3' motif.

In more preferred embodiments, the CpG of the invention is an oligodeoxynucleotide which comprises one or more CpG motifs, more preferably two or more CpG motifs, has preferably a length 18-25 nucleotides, is a fully thio-modified sequences, and the CpG-ODN comprises two or more copies of 5'-TCGT-3' motif or 5'-TCGTCGT-3' motif, preferably the CpG of the invention includes or consists of a CpG of class B, wherein preferably the CpG comprises two or more copies of 5'-TCGT-3' motif or 5'-TCGTCGT-3' motif.

In particular, the CpG has a sequence selected from:
5'-TCG TCG TTT TGT CGT TTT GTC GTT-3' (SEQ ID NO: 1),
5'-TCG TTC GTT CGT TCG TTC GTT-3' (SEQ ID NO: 2),
5'-TCG TTC GTT CGT TCG TTC GTT CGT T-3' (SEQ ID NO: 3),
5'-TCG TCG TCG TCG TCG TCG TCG-3' (SEQ ID NO: 4),
5'-TCC ATG ACG TTC CTG ACG TT-3' (SEQ ID NO: 5),
5'-TGA CTG TGA ACG TTC GAG ATG A-3' (SEQ ID NO: 6),
5'-TCG TTC GTT CGT TCG TTC GTT (SEQ ID NO: 7),
5'-TCG TCG TCG TCG TCG TCG TCG (SEQ ID NO: 8),
5'-TCG ACG TTC GTC GTT CGT CGT TC (SEQ ID NO: 9),
5'-TCC ATG ACG TTC CTG ATG CT (SEQ ID NO: 10), and
5'-TGT CGT CGT CGT TTG TCG TTT GTC GTT (SEQ ID NO: 11)
preferably SEQ ID NO: 1 (DN 2006) or SEQ ID NO: 5 (CpG-ODN 1826).

In very preferred embodiments, the CpG of the invention comprises or consists of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1-4 nucleotides is/are substituted, inserted, or deleted, and the variant comprises at least one, preferably at least two CpG motifs. In a more preferred embodiments, the CpG of the invention is an oligodeoxynucleotide which has preferably a length 18-25 nucleotides and is fully thio-modified, wherein the CpG of the invention comprises or consists of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1-4 nucleotides is/are substituted, inserted, or deleted, and the variant comprises at least one, preferably at least two CpG motifs. Preferably, the oligonucleotide sequence is selected from the group consisting of SEQ ID NO: 1-11.

In very preferred embodiments, the CpG of the invention comprises or consists of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1-3 nucleotides is/are substituted, inserted, or deleted, and the variant comprises at least one, preferably at least two CpG motifs. In a more preferred embodiments, the CpG of the invention is an oligodeoxynucleotide which has preferably a length 18-25 nucleotides and is fully thio-modified, wherein the CpG of the invention comprises or consists of an oligonucleotide sequence selected from the group consisting of SEQ ID NO: 1-11, ODN1018, K3-CpG, CPG T1, T2, T3, ODNBW006, ODN D-SL01, ODN2006, and ODN684; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1-3 nucleotides is/are substituted, inserted, or deleted, and the variant comprises at least one, preferably at least two CpG motifs. Preferably, the oligonucleotide sequence is selected from the group consisting of SEQ ID NO: 1-11.

In very preferred embodiments, the CpG of the invention comprises or consists of an oligonucleotide sequence selected from the group consisting of SEQ ID NO: 1-11, ODN 1826, ODN1018, K3-CpG, CPG T1, T2, T3, ODNBW006, ODN D-SL01, ODN2006, and ODN684; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1 or 2 nucleotides is/are substituted, inserted, or deleted, and the variant comprises at least one, preferably at least two CpG motifs. In a more preferred embodiments, the CpG of the invention is an oligodeoxynucleotide which has preferably a length 18-25 nucleotides and is fully thio-modified, wherein the CpG of the invention comprises or consists of an oligonucleotide sequence selected from the group consisting of SEQ ID NO: 1-11, ODN 1826, ODN1018, K3-CpG, CPG T1, T2, T3, ODNBW006, ODN D-SL01, ODN2006, and ODN684; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1 or 2 nucleotides is/are substituted, inserted, or deleted, and the variant comprises at least one, preferably at least two CpG motifs. Preferably, the oligonucleotide sequence is selected from the group consisting of SEQ ID NO: 1-11.

In very preferred embodiments, the CpG of the invention comprises or consists of an oligonucleotide sequence selected from the group consisting of SEQ ID NO: 1-11, ODN 1826, ODN1018, K3-CpG, CPG T1, T2, T3, ODNBW006, ODN D-SL01, ODN2006, and ODN684, preferably the CpG of the invention comprises or consists of an oligonucleotide sequence selected from the group consisting of SEQ ID NO: 1-11.

In a more preferred embodiments, the CpG of the invention is an oligodeoxynucleotide which has preferably a length 18-25 nucleotides and is fully thio-modified, wherein the CpG of the invention comprises or consists of an oligonucleotide sequence selected from the group consisting of SEQ ID NO: 1-11, ODN 1826, ODN1018, K3-CpG, CPG T1, T2, T3, ODNBW006, ODN D-SL01, ODN2006, and ODN684; preferably the CpG of the invention comprises or consists of an oligonucleotide sequence selected from the group consisting of SEQ ID NO: 1-11.

In very preferred embodiments, the CpG of the invention comprises or consists of an oligonucleotide sequence selected from the group consisting of SEQ ID NO: 1, SEQ ID NO: 5, ODN 1826, and ODN2006; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1, 2, 3 or 4, preferably 1 or 2 nucleotide(s) is/are substituted, inserted, or deleted, and the variant comprises at least one, preferably at least two CpG motifs. In a more preferred embodiments, the CpG of the invention is an oligodeoxynucleotide which has preferably a length 18-25 nucleotides and is fully thio-modified, wherein the CpG of the invention comprises or consists of an oligonucleotide sequence selected from the group consisting of SEQ ID NO: 1, SEQ ID NO: 5, ODN 1826, and ODN2006; or the CpG oligonucleotide comprises or consists of a variant of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1, 2, 3 or 4, preferably 1 or 2 nucleotide(s) is/are substituted, inserted, or deleted, and the variant comprises at least one, preferably at least two CpG motifs. Preferably, the oligonucleotide sequence is selected from the group consisting of SEQ ID NO: 1-11.

Preferably, in the variants of the oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, the inserted, substituted or deleted nucleotides are outside the CpG motifs, in other words in the variants the CpG motifs are preferably unmodified.

In very preferred embodiments, the CpG of the invention comprises or consists of an oligonucleotide sequence selected from the group consisting of SEQ ID NO: 1, SEQ ID NO: 5, ODN 1826, and ODN2006. In a more preferred embodiments, the CpG of the invention is an oligodeoxynucleotide which has preferably a length 18-25 nucleotides and is fully thio-modified, wherein the CpG of the invention comprises or consists of an oligonucleotide sequence selected from the group consisting of SEQ ID NO: 1, SEQ ID NO: 5, ODN 1826, and ODN2006.

In very preferred embodiments, the CpG of the invention comprises or consists of a CpG of SEQ ID NO: 1 or 5.

### IL-7 and IL7R

The composition of the invention comprises an Interleukin-7 (IL-7) antagonist.

Interleukin-7 (IL-7) is a soluble globular single-chain protein or glycoprotein binding to IL-7 receptor and encoded by the IL7 gene and produced predominantly by epithelial cells, especially keratinocytes and thymic epithelial cells. IL-7 is a non-redundant growth, differentiation and survival factor for T and B cells in the mouse and of T cells in humans. Accession numbers of certain IL-7 genes and proteins are as follows: human, M29048; P13232 IL7_HUMAN; mouse, AH_001973; P10168 IL7_MOUSE. Also encompassed by the term IL-7 are homologues or mimetics of IL-7 which possess qualitative biological activity in common with the full-length mature activated IL-7.

As used herein the term "Interleukin-7 receptor" (herein synonymously mentioned as IL7R) preferably refers to a cell surface-expressed heterodimer comprising IL-7 receptor-α chain (IL-7Rα, CD127; P16871 IL7RA_HUMAN; P16872 IL7RA_MOUSE) and common-γ chain (γc, CD132; P31785 IL2RG_HUMAN; P34902 IL2RG_MOUSE), as well as variants, isoforms and N-glycosylated forms of CD127 or CD132, preferably as specified on UniProt. For example, the term CD127 includes four isoforms of Swissprot accession numbers P16871-1 to P16871-4. The common-γ chain receptor is shared with various cytokines, including Interleukin-2, -4, -7, -9, and -15. Interleukin-7 receptor is expressed on the surface of various cell types, including naive and memory T cells. IL-7 receptors are known and described, e.g., in U.S. Patent No. 5,264,416. Also encompassed by the term IL7R are homologues or mimetics of IL-7R which possess qualitative biological activity in common with IL-7R.

Binding of IL-7 to the IL-7R activates multiple signaling pathways including the activation of JAK kinases 1 and 3 leading to the phosphorylation and activation of Stats, such as STAT5, and inhibition of IL-7 mediated TH17 expansion.

### IL-7 antagonist

Preferably, the IL-7 antagonist is an IL-7 receptor (IL7R), or the IL-7 antagonist binds to an IL7R or the IL-7 antagonist binds to IL-7.

The term "IL-7 antagonist" as used herein refers to any compound or active agent which inhibits or blocks the activity of IL-7 or IL7R or the IL-7 signaling pathway, including but not limited to compounds that bind to IL-7 receptors and IL-7 itself. The term "IL-7 antagonist" includes also antagonists that inhibit or block the IL-7 signaling pathway upstream or downstream of IL-7 and IL7R, preferably at or downstream of IL7R.

In certain embodiments, the IL-7 antagonist includes compounds that inhibit or block binding to IL7R, and/or compounds that do not compete with IL-7 in IL7R binding or do not block IL7R binding. In certain embodiments, the IL-7 antagonist includes compounds that do not have an antagonistic effect or agonistic effect on the IL-7 receptor, but present cytotoxic activity against CD127 positive cells. The term "IL-7 antagonist" as used herein also includes compounds that do not inhibit human IL-7 induced phosphorylation of STAT5 in cells expressing the IL7-R; do not inhibit human TSLP-stimulated secretion of TARC in cells expressing the TSLP-R; do not activate CD127, especially human CD127; do not inhibit CD127, especially human CD127; do not increase human IL-7 induced phosphorylation of STAT5 in cells expressing the IL7-R; and/or do not increase human TSLP-stimulated secretion of TARC in cells expressing the TSLP-R.

The term "IL-7 antagonist" as used herein also includes compounds that do not compete with IL-7 for binding to IL-7R.

Preferably, the IL-7 antagonist is an IL7R antagonist. Preferably the IL7R antagonist binds to the IL-7 receptors, thereby activity of IL7R can be blocked or inhibited. More preferably, the IL-7 receptor antagonist binds to IL-7Rα, thereby IL-7Rα signaling can be blocked or inhibited. The antagonist properties as disclosed in the present invention may be in particular antagonism toward IL-7R signaling induced by IL-7, especially human IL-7. An antagonist of IL-7R signaling induced by IL-7 can be identified by measuring the inhibition of STAT5 phosphorylation by methods known in the art.

The term "binding" as used herein, typically refers to association (e.g., non-covalent or covalent) between the IL-7 receptor antagonist and its binding partner, such as the IL7R. The term "binding" according to the invention preferably relates to a specific binding. "Specific binding" means that an IL-7 receptor antagonist binds stronger to a binding partner for which it is specific compared to the binding to another non-specific binding partner. An IL-7 receptor antagonist is considered to bind specifically to its binding partner if it has a dissociation constant K_{D} of 1 µM or less, or preferably 100 nM or less, more preferably 10 nM or less, even more preferably 1 nM or less, even more preferably 0,5 nM, 0,3 nM, or 0,1 nM or less.

The IL-7 antagonist can be a nucleic acid-based antagonist, a protein-based antagonist or a small molecule antagonist, preferably a protein-based antagonist. The nucleic acid-based antagonist may be a siRNA molecule or an antisense construct.

In certain other embodiments, the IL-7 antagonist binds to IL7R. Preferably, the IL-7 antagonist binding to IL7R binds to an alpha chain of IL7R (CD127).

In preferred embodiments, the IL-7 antagonist binding to IL7R is an IL7R binding protein. More preferably, the IL-7 antagonist binding to IL7R or IL-7 is an antibody, or an antigen binding fragment thereof. The IL-7 antagonist or IL7R binding protein is preferably an anti-IL-7 receptor antibody (IL7R antibody) or an anti-IL-7 antibody (IL-7 antibody), or an antigen binding fragment thereof binding to IL7R (IL7R binding fragment) or IL-7 (IL-7 binding fragment).

In preferred embodiments, the IL-7 antagonist or IL7R antagonist is a monoclonal antibody, preferably an IL-7 antibody or IL7R antibody, more preferably an IL7R antibody.

### Antibody

The term "antibody" is used herein body is used interchangeably with the term "immunoglobulin" and includes monoclonal antibodies, polyclonal antibodies, fully-human antibodies, intact antibodies, fragments of antibodies, as long as the fragment exhibit the desired antigen-binding activity, intact antibodies and fragments that have been mutated either in their constant and/or variable region, e.g., chimeric, partially or fully humanized antibodies, antibodies with a desired trait, e.g., enhanced binding and/or reduced FcR binding, heteroconjugate antibodies and multi-specific antibodies (e.g., bispecific antibodies), recombinant antibodies, especially recombinant human antibodies, antigen-binding fragments of recombinant antibodies, heterologous antibodies, heterohybrid antibodies, or antibodies displayed upon the surface of a phage or displayed upon the surface of a cell (e.g., a chimeric antigen receptor T cell).

The term "antibody fragment", "fragment of an antibody" or "antigen binding fragment" refers to a portion of an antibody or antibody chain comprising fewer amino acid residues than an intact or complete antibody. Fragments can be obtained via chemical or enzymatic treatment of an intact or complete antibody or antibody chain or recombinant means. Exemplary fragments include Fab, Fab', F(ab')2, Fabc, Fd, Fd', dAb, V-NARs, Fv, immunoglobulin chain variable domains such as VLs, VHHs and VHs, isolated CDR regions or isolated CDR regions in a functional framework, single chains, dsFv, single-chain antibodies, e.g., scFv, single domain antibodies, antigen-binding fragments combined together to obtain multivalent antigen-binding fragments, such as diabodies, tribodies or tetrabodies; linear antibodies comprising a pair of tandem Fd segments (VH-CH1-V-CH1) which, together with complementary light chain polypeptides, form a pair of antigen binding regions. Preferably, the fragment retains the ability to bind to an antigen, e.g., IL-7 or IL7R. Antibodies are grouped into different isotypes based on the heavy chain they possess. The antibody can be selected from the group consisting of IgA, IgD, IgE, IgG, and IgM antibodies. The IgG antibody can be selected from the group consisting of an IgG1, IgG2a or IgG2b, IgG3 or IgG4 antibody. The antigen-binding fragments of the antibody may be obtained starting from the antibody, especially by using enzymatic digestion according to well-known techniques including papain or pepsin digestion, or using any appropriate cleavage technique. They may be alternatively expressed in host cells modified by recombination with nucleic acid sequences encoding the amino acid sequence of said fragments, or may be synthesized, especially chemically synthesized. Accordingly, the antibodies of the invention, including the modified antibodies, and the antigen- binding fragments of the antibodies can also be prepared by classical genetic engineering techniques, such as those described by Sambrook et al. (Molecular Cloning, A Laboratory Manual, 2nd Ed., Cold Spring Harbor Laboratory Press, Cold Spring Harbor, N. Y., (1989), and updated versions). Preferably, the antibody of the invention is a polyclonal or monoclonal antibody, more preferably a monoclonal antibody. As used herein, the term "monoclonal antibody" refers to an antibody derived from a clonal population of antibody producing cells (e.g., B lymphocytes or B cells) which is homogeneous in structure and antigen specificity. The term "polyclonal antibody" refers to a plurality of antibodies originating from different clonal populations of antibody producing cells which are heterogeneous in their structure and epitope specificity, but which recognize a common antigen. Monoclonal and polyclonal antibodies may exist within body fluids, as crude preparations, or may be purified, as described herein.

Preferably, the antibody of the invention is a human, partially or fully humanized antibody. Preferably the antibody is Fc-disabled.

### IL7R antibody and IL-7 antibody

In certain embodiments, the IL7R antibody is selected from an IL7R antibody known in the art, such as antibodies described, for example without limitation, in any of the following published patents or patent applications: WO2011/104687 (including, for example, but without limitation, any of the antibodies listed in Table 1), US9150653B2, US5194375, WO2011094259, WO2016059512A1, WO2013056984, WO2006052660, WO2015189302A1, WO2017149394, WO2020077190, WO2021236691, WO2017062748A1, and WO2010017468, GSK2618960, PF-06342674 (RN168), and OSE-703.

In preferred embodiments, the IL7R antagonist binds to an alpha chain of IL7R (CD127). In preferred embodiments, the IL7R binding protein, IL7R antibody, or the antigen binding fragment thereof, binds to an IL7R alpha chain (CD127). Preferably, the IL7R antagonist is an anti-CD127 antibody (CD127 antibody).

In certain other embodiments, the IL-7 antagonist is an exogenous IL-7 receptor, for example a soluble IL-7 receptor, administered to bind endogenous IL-7 and make it unavailable or less available for binding to the endogenous IL-7 receptor. ILR receptors are known in the art and described, e.g., in U.S. Patent No. 5,264,416. In certain other embodiments, the IL-7 antagonist binds to IL-7. More preferably, the IL-7 antagonist binding to IL-7 is an IL-7 binding protein. Again more preferably, the IL-7 antagonist or IL-7 binding protein is an anti-IL-7 antibody (IL-7 antibody), or an antigen binding fragment thereof binding to IL-7 (IL-7 binding fragment). In certain embodiments, the IL-7 antagonist is a monoclonal IL-7 antibody. Such antibodies are known and described, e.g., in U.S. Patent No. 5,714,585.

The term "antigen binding fragment thereof, binding to" IL-7 or IL7R of an IL-7 or IL7R antibody refers to a fragment of an antibody or antigen binding protein, wherein the fragment retains the ability to bind to IL-7 or IL7R. In other embodiments, the fragment retains the ability to bind to IL7R, to inhibit IL-7 mediated TH17 expansion, IL-7 induced IL-17 production by TH17 cells, IL-7 induced IFN-γ production by TH17 cells, IL7-induced internalization of CD127 and/or IL-7 receptor mediated STAT-5 phosphorylation and/or to disrupt the binding of CD127 to the yc common chain of cytokine receptors. Preferably the antigen binding fragment is a portion of the IL-7 or IL7R antibody that binds to the alpha chain of the IL-7 receptor for IL-7, preferably in its native form. Preferably, the antigen-binding fragments have a similar binding affinity as the corresponding full antibodies. However, antigen-binding fragment that have a reduced antigen-binding affinity with respect to corresponding full antibodies are also encompassed within the invention. Preferably, the antigen-binding fragments comprise hypervariable domains designated CDRs (Complementary Determining Regions) or the recognition site for the antigen, e.g., IL-7Ra of human IL-7, of the full antibody.

Monoclonal antibodies may be recombinant monoclonal antibodies produced according to the methods disclosed in U.S. Pat. No. 4,474,893 or U.S. Pat. No. 4,816,567. The antibodies may also be chemically constructed by specific antibodies made according to the method disclosed in U.S. Pat. No. 4,676,980. Monoclonal antibody active agents may be chimeric antibodies produced in accordance with known techniques, cf. e.g., U.S Patent No. 6,808,901. For example, chimeric monoclonal antibodies may be complementarity determining region-grafted antibodies (or "CDR-grafted antibodies") produced in accordance with known techniques. Monoclonal antibodies can be humanized by methods known in the art, e.g., MAbs with a desired binding specificity can be commercially humanized (Scotgene, Scotland; Oxford Molecular, Palo Alto, Calif.).

Monoclonal antibodies against CD127 having antagonist properties toward IL7R alpha chain have been disclosed in WO2010/017468 and their humanized versions in WO2011/094259 with a view to treat autoimmune diseases like multiple sclerosis. The described antibodies are said to be antagonist for IL-7 binding to its receptor, and active against TH1 and TH1 cells expansion and survival which were said to require IL-7 interaction with their CD127 receptor. Monoclonal antibody Fab fragment active agents may be produced in Escherichia coli by recombinant techniques known to those skilled in the art (e.g., W. Huse, Science 246, 1275-81 (1989)).

Preferably, the IL-7 binding protein, IL7R binding protein, IL7R antibody, IL-7 antibody, or an antigen binding fragment thereof binds specifically to IL7R or IL7, preferably with a dissociation constant K_{D} of at least 1 µM or less, or preferably 100 nM or less, more preferably 20 nM or less, even more preferably 10 nM or less, even more preferably 1 nM or less, even more preferably 0,5 nM, 0,3 nM, or 0,1 nM or less. Preferably, the dissociation constant K_{D} is measured by surface plasmon resonance (BIAcore).

In one embodiment, the IL7R binding protein, CD127 binding protein, IL7R antibody, or CD127 antibody, or a binding fragment thereof, inhibits the binding of IL-7 to IL7R. Binding to IL7R, preferably to CD127, preferably inhibits the biological function of IL7R and CD127, respectively, such as inhibition of IL-7 mediated TH17 expansion, IL-7 induced IL-17 production by TH17 cells, IL-7 induced IFN-γ production by TH17 cells and/or IL-7 receptor mediated STAT-5 phosphorylation. Preferably, the IL7R or CD127 binding molecule, or the antigen binding fragment thereof, has antagonist properties for IL-7/IL7R interaction, may present cytotoxic activity against CD127 positive cells and do not increase the maturation of dendritic cells (DCs) induced by TSLP, a cytokine also using CD127 as part of its receptor. Alternatively, or in addition, these antibodies do not induce the internalization of CD127 and/or inhibit the IL7-induced internalization of CD127.

In preferred embodiments, the IL7R binding protein, CD127 binding protein, IL7R antibody, or CD127 antibody is selected from the group consisting of BD, 9B7, 6C5, 6A3, R34.34, GR34 (WO2010017468A1), N13B2, N13B2-h1, N13B2-h2, N13B2-h3, UniProt P20759, C1 GM (WO2011104687, especially Table 1), GSK2618960, 1A11, especially 1A11.H3L4, BPC4401, BPC4398, BPC1142, BPC4399, BPC4402, BPC4403, and BPC1142 (WO2011/094259), antibodies MD707-1, MD707-2, MD707-3, MD707-4, MD707-5, MD707-6, MD707-9, MD707-12, and MD707-13 (WO2013/056984), or the IL7R binding protein, CD127 binding protein, IL7R antibody, or CD127 antibody is a humanized or chimeric version thereof, or an antigen-binding fragments thereof.

Preferably, the IL7R or CD127 binding protein comprises a heavy chain and/or light chain variable region of an IL7R or CD127 binding protein selected from the group consisting of BD, 9B7, 6C5, 6A3, R34.34, GR34 (WO2010017468A1), N13B2, N13B2-h1, N13B2-h2, N13B2-h3, UniProt P20759, C1 GM (WO2011104687, especially Table 1), GSK2618960, 1A11, especially 1A11.H3L4, BPC4401, BPC4398, BPC1142, BPC4399, BPC4402, BPC4403, and BPC1142 (WO2011/094259), antibodies MD707-1, MD707-2, MD707-3, MD707-4, MD707-5, MD707-6, MD707-9, MD707-12, and MD707-13 (WO2013/056984), or a humanized or chimeric version thereof.

Preferably, the IL7R or CD127 binding protein comprises one, two or three of the heavy chain complementarity determining regions CDRH 1, 2, and 3, and/or one, two or three of the light chain complementarity determining regions CDRL 1, 2, and 3 of an IL7R or CD127 binding protein selected from the group consisting of BD, 9B7, 6C5, 6A3, R34.34, GR34 (WO2010017468A1), N13B2, N13B2-h1, N13B2-h2, N13B2-h3, UniProt P20759, C1 GM (WO2011104687, especially Table 1), GSK2618960, 1A11, especially 1A11.H3L4, BPC4401, BPC4398, BPC1142, BPC4399, BPC4402, BPC4403, and BPC1142 (WO2011/094259), antibodies MD707-1 , MD707-2, MD707-3, MD707-4, MD707-5, MD707-6, MD707-9, MD707-12, and MD707-13 (WO2013/056984), or a humanized or chimeric version thereof.

The IL7R antigen binding protein may preferably comprise a heavy chain variable region selected from the group consisting of 1A11.H0 VH to 1A11.H7 VH, or 1, 2 or 3 CDRHs thereof (WO2011/094259). In a preferred embodiment, the antigen binding protein comprises the heavy chain variable region of 1A11.H3 VH, or 1, 2 or 3 CDRHs thereof. The antigen binding protein may preferably comprise a light chain variable region 1A11.L0 Vκ to 1A11.L9 Vκ, or 1, 2 or 3 CDRLs thereof. In an embodiment, the antigen binding protein comprises a light chain variable region of 1A11.L4 Vκ, or 1, 2 or 3 CDRLs thereof. In a particularly preferred embodiment, the antigen binding protein comprises the heavy chain variable region 1A11.H3 VH and/or the light chain variable region of 1A11.L4 Vκ. In another particularly preferred embodiment, the antigen binding protein comprises the 1, 2, or 3 CDRHs of the heavy chain variable region 1A11.H3 VH and/or 1, 2, or 3 CDRHs of the light chain variable region of 1A11.L4 Vκ.

Throughout this specification, the terms "CDR", "CDRL1 ", "CDRL2", "CDRL3", "CDRH 1 ", "CDRH2", "CDRH3" follow the Kabat numbering system, as set forth in Kabat et al; Sequences of proteins of Immunological Interest NIH, 1987.

In a very preferred embodiment, the IL7R antagonist is a human or humanized immunoglobulin G1 (IgG1) monoclonal antibody that binds to the α-chain of the heterodimeric IL7R, such as GSK2618960. Preferably the antibody is Fc-disabled.

In a preferred embodiment, the IL-R antagonist is a chimeric molecule comprising an IL7R or IL-7 antibody or an antigen binding fragment thereof as defined herein. In a particular embodiment, the chimeric molecule is a CAR molecule which comprises at least 2, preferably at least 3 signaling domains.

In a preferred embodiment, the IL-R antagonist is cell comprising an IL7R or IL-7 antibody or an antigen binding fragment thereof as defined herein, wherein the antibody or antigen-binding fragment thereof is exposed as an ectodomain at the surface of the cell. The cell may be a T cell, such as an autologous T cell of a patient or an allogenic T cell.

### Kinase inhibitor

The term "kinase inhibitor" preferably refers to a molecule that antagonizes, blocks, or inhibits phosphorylation-dependent cell signaling and/or growth pathways in a cell. Kinase inhibitors may be naturally occurring or synthetic and include small molecules. Kinase inhibitors have the ability to selectively block or inhibit the activity of the target kinase molecules. Kinases fall into two broad classes, characterized with respect to substrate specificity: serine/threonine-specific, and tyrosine-specific. Kinase inhibitors preferably bind to the inactive or active conformation of the kinase and thus blocks its catalytic activity. Non-limiting examples of target kinase molecules can be selected from the group consisting of CSF1R, ABL, Bcr-Abl, c-KIT, FLT3, and PDGFR-β. Examples of kinase inhibitors include imatinib, sorafenib (Nexavar^{®}), Sutent^{®}, dasatinib, Dasatinib^{™}, Zactima^{™}, Tykerb^{™} and STI571.

In a certain embodiment, the kinase inhibitor is a tyrosine kinase inhibitor. The term "tyrosine kinase inhibitor" (mentioned synonymously herein as TKI) preferably refers to a molecule that antagonizes, blocks, or inhibits tyrosine kinase. Preferably the TKI is a small molecule inhibitor of tyrosine kinases. TKIs can disrupt the signal transduction pathways of protein kinases. TKIs are preferably enzymes capable of phosphorylating the amino acid tyrosine in another protein, which leads to conformational changes and activation, inhibition, or blocking of that protein. The term TKI as used herein comprises cell-surface receptors or receptor tyrosine kinases, which penetrate the plasma membrane and possess a kinase domain in the cytoplasmic region, such as the receptors for many growth factors; non-receptor tyrosine kinases and dual-specificity kinases (Tyrosine Kinase Inhibitors, Robert J. Thomson, StatPearls Publishing, 2024). Non-receptor tyrosine kinases, such as Src family tyrosine kinases and Janus kinases, reside in the cytoplasm and serve as intracellular signal transducers, preferably ensuring that binding of a transmembrane receptor for cytokines, growth factors, and so on, by its cognate ligand leads, by way of a sequence of activation events, to the initiation of transcription in the nucleus. Dual-specificity kinases can phosphorylate serine, threonine, and tyrosine residues. Most preferably, the TKI is imatinib.

In a certain embodiment, the kinase inhibitor or tyrosine kinase inhibitor is a Bcr-Abl tyrosine-kinase inhibitor. The term "Bcr-Abl tyrosine-kinase inhibitor" preferably refers to a molecule that antagonizes, blocks, or inhibits the enzyme Bcr-Abl tyrosine-kinase. Reciprocal translocation between chromosomes 9 and 22 produces the bcr-abl oncogene that when translated, yields the p210 BCR-ABL. This protein has constitutive tyrosine kinase activity that activates numerous downstream pathways that ultimately produces uncontrolled myeloid proliferation.

The kinase inhibitors, tyrosine kinase inhibitors, or Bcr-Abl tyrosine-kinase inhibitors can be selected from the non-limiting group consisting of Imatinib, Nilotinib, Dasatinib, Bosutinib, Ponatinib, Bafetinib, Asciminib, 1,3,4 thiadiazole derivatives, Substance 14, and combinations thereof.

Resistance to Bcr-Abl tyrosine-kinase inhibitors is preferably related to Bcr-Abl dependent mechanisms of resistance including Bcr-Abl duplication, Bcr-Abl mutation, T315I mutation, and P-loop mutations; and/or to Bcr-Abl independent mechanisms of resistance including drug efflux caused by P-glycoproteins, drug import by organic cation transporter 1, and alternative signaling pathway activation.

### Treatment or prevention

In a further aspect, the invention relates to the composition of the invention for use in treating or preventing leukemia lymphoblastic leukemia. In another aspect, the invention relates to a method for treating or preventing leukemia, preferably lymphoblastic leukemia, said method comprises administering to a patient suffering leukemia a therapeutically effective dose of the composition of the invention.

Preferably, the leukemia is acute leukemia or chronic leukemia, preferably acute lymphoblastic leukemia (also called acute lymphocytic leukemia, ALL) or chronic lymphatic leukemia (also called chronic lymphocytic or chronic lymphoblastic leukemia or CLL), again more preferably acute lymphoblastic leukemia (ALL). The terms lymphatic, lymphocytic and lymphoblastic are used interchangeably herein.

In certain embodiments, ALL is Ph-negative, Ph-positive or Ph-like ALL, preferably Ph-positive or Ph-like ALL, more preferably Ph-positive ALL.

The term "acute lymphoblastic leukemia" (ALL) comprises (i) T-ALL encompassing T-ALL not otherwise specified, and the early T cell precursor acute lymphoblastic leukemia/lymphoma (ETP-ALL), and (ii) B-ALL (or BCP-ALL, which is interchangeably used herein with the term B-ALL). Preferably, ALL is B-ALL.

In certain embodiments, ALL is Ph-negative BCP-ALL, Ph-positive or Ph-like ALL BCP-ALL, preferably Ph-positive or Ph-like ALL, more preferably Ph-positive ALL BCP-ALL.

The term ALL, as used herein, preferably refers to a hematological malignancy of the lymphoid line of blood cells. ALL arises from the transformation and uncontrolled proliferation of B cell precursor or T lymphoid progenitors and thus includes B-cell precursor ALL (BCP-ALL) and T cell ALL (T-ALL). The B or T lymphoid pro-genitors (lymphoblasts) are halted at any of several immature stages of lymphoid differentiation proliferating and replacing normal hematopoietic cells of the bone marrow and peripheral blood. ALL presents as blast infiltration of the bone marrow and additional peripheral organs such as liver, spleen and lymph nodes, leading to elevated white blood cell (WBC) count, reduction in red blood cell and platelet (pancytopenia) counts and central nervous system involvement, which can be a site of disease relapse. At diagnosis, BCP-ALL can rarely present as a lymphoblastic lymphoma (LBL); alternatively, Burkitt's lymphoma can occasionally present with stage IV marrow involvement, consistent with leukemia. T-ALL often presents with lymphomatous features, such as nodal or extra-nodal onset, including thymic enlargement.

The 5th edition of the WHO Classification of Hematolymphoid Tumors (WHO-HAEM5 2022) upheld the distinction of two histological subgroups in ALL, while introducing a novel nomenclature focusing on the definition of molecular events, such as BCR::ABL1 fusion rather than on the corresponding cytogenetic alterations, such as t(9;22)(q34.1;q11.2) and acknowledged the provisional entity of BCP-ALL with BCR::ABL1-like features as distinct categories. A second panel of experts from the International Consensus Classification (ICC) formulated a slightly different classification of ALL. This classification includes provisional entities and new subtypes not covered in WHO-HAEM5, emphasizing driver structural lesions or distinctive gene expression signatures (Pagliaro L et al. (Acute lymphoblastic leukemia, Nat Rev Dis Primers 10, 41 (2024), https://doi.org/10.1038/s41572-024-00525-x). The term ALL as used herein includes all subtypes of ALL, especially those described in Pagliaro et al, 2024.

In certain embodiments, ALL is BCP-ALL as defined in Pagliaro L et al. (op. cit.), especially in Table 1 and supplementary Table 1. In certain embodiments, ALL is B-lymphoblastic leukemia with BCR::ABL1 fusion and B-lymphoblastic leukemia/lymphoma with BCR::ABL 1-like features. In certain embodiments, ALL is B-lymphoblastic leukemia with BCR::ABL1 fusion or B-lymphoblastic leukemia/lymphoma with BCR::ABL 1-like features. In certain embodiments, ALL is B-lymphoblastic leukemia with BCR::ABL1 fusion. In certain embodiments, ALL is or B-lymphoblastic leukemia with BCR::ABL 1-like features.

ALL can be Ph+ ALL characterized by the presence of a fusion gene encoding Bcr-Abl (Bcr-Abl+) or by the presence of a Philadelphia chromosome (Philadelphia chromosome-positive ALL) or by the presence of a BCR::ABL1-like transcriptional profile (as defined in Pagliaro L et al., 2024, op. cit.). Preferably, ALL is Ph+ ALL characterized by the presence of a fusion gene encoding Bcr-Abl or a Philadelphia chromosome or both. In a certain embodiment, ALL is Ph+ ALL characterized by the presence of a Philadelphia chromosome. In a certain embodiment, ALL is Ph+ ALL characterized by the presence of a fusion gene encoding Bcr-Abl.

In certain embodiments, Ph+ ALL comprises or is Philadelphia chromosome-positive (Ph+) like acute lymphoblastic leukemia (Ph+ like ALL), Bcr-Abl+ like ALL, or BCR::ABL1-like ALL. Preferably, "BCR::ABL1-like ALL", also referred to as Ph-like ALL, refers to ALL characterized by a transcriptional profile similar to that of Ph-positive ALL, but it lacks the BCR::ABL1 fusion gene.

In preferred embodiments, the patient is resistant to treatment with kinase inhibitors, preferably tyrosine kinase inhibitors, more preferably Bcr-Abl tyrosine-kinase inhibitors.

In some embodiments, the patient has circulating malignant cells, preferably expressing IL7R. In some embodiments, the composition is used to treat patients who relapsed following therapy using tyrosine kinase inhibitors. Preferably, in the relapsed patients the circulating malignant cells are expressing IL7R.

The term "Philadelphia chromosome-positive acute lymphoblastic leukemia" (Ph+ ALL) as used herein, refers to an aggressive form of ALL. Ph+ ALL comprises forms of ALL, wherein the BCR-ABL1 oncogene is expressed as the result of a reciprocal translocation between the ABL1 oncogene, or part thereof, on the long arm of chromosome 9 and a breakpoint cluster region (BCR), or part thereof, on the long arm of chromosome 22, also called Philadelphia-translocation t(9;22)(q34;q11.2). The resulting fusion gene, BCR-ABL, encodes an oncogenic protein with constitutively active tyrosine kinase activity. There are two main breakpoint regions within the BCR gene, namely major or minor breakpoint cluster region (M-BCR or m-BCR). M-BCR lies between exons 12 and 16, producing the larger p210 oncoprotein, while the m-BCR yields a smaller p190 product that retains only the first exon of BCR. A third rare breakpoint between exons 19 and 20 has also been described, giving rise to the p230 fusion protein (Dalle et al., Leukemia (2024) 38:1876-1884; https://doi.org/10.1038/s41375-024-02319-2).

The term "subject" or "patient" preferably refers to a mammal, such as a human. Mammals generally include, but are not limited to, humans, non-human primates, rats, mice, cats, dogs, horses, cattle, cows, pigs, sheep and rabbits.

As used herein the terms "treating" and "treatment" refer to any and all uses intended to obtain a beneficial or desired result, to remedy a condition or symptoms, prevent the establishment of a condition or disease, alleviate symptoms or otherwise prevent, hinder, retard, or reverse the progression of a condition or disease or other undesirable symptoms in any way whatsoever.

As used herein the term "effective amount" includes within its meaning a non-toxic but sufficient amount of an agent or compound to provide the desired effect. The exact amount required will vary from subject to subject depending on factors such as the species being treated, the age and general condition of the subject, the particular agent being administered and the mode of administration and so forth. Thus, it is not possible to specify an exact "effective amount". However, for any given case, an appropriate "effective amount" may be determined by one of ordinary skill in the art using only routine experimentation.

The term "pharmaceutically acceptable" preferably refers to a material that does not interfere with the effectiveness of a compound according to the invention, and that is compatible with a biological system such as a cell, cell culture, tissue, or organism. Preferably, the biological system is a living organism, such as a vertebrate. The term a "pharmaceutically effective amount" generally refers to an amount sufficient to affect a desired biological effect, such as a beneficial result. Thus, a "pharmaceutically effective amount" will depend upon the context in which it is being administered. A pharmaceutically effective amount may be administered in one or more prophylactic or therapeutic administrations.

The composition of the invention is preferably prepared for storage by mixing the active compound having the desired degree of purity with optional physiologically acceptable carriers, excipients or stabilizers (Remington's Pharmaceutical Sciences, 23rd edition), e.g., in the form of lyophilized formulations or aqueous solutions.

Administration of the composition of the invention can be by any suitable route, including, without limitation, parenteral, oral, intratumoral, sublingual, transdermal, topical, intranasal, aerosol, intraocular, intratracheal, intrarectal, mucosal, vaginal, by gene gun, dermal patch or in eye drop or mouthwash form. Administration can be carried out using known procedures at dosages and for periods of time effective to reduce symptoms or surrogate markers of the disease.

The IL-7 antagonist anti-IL7R antibodies can be administered prior to, simultaneously with, or subsequent to the administration of the TLR9 agonist.

In certain preferred embodiments, the composition of the invention is co-administered or administered in combination with another agent, including without limitation further antibodies, cytotoxic agents, allergens, kinase inhibitors, antisense oligonucleotides, siRNA, aptamers, ribozymes, targeted therapies, peptides, proteins, antibiotics, gene therapy vectors, DNA vaccines, and/or adjuvants to enhance the specificity or magnitude of the immune response.

As used herein, the terms "antagonize" or "inhibit" refers to reduction of biological activity of the bound molecule, e.g., the antigen. In certain embodiments, the term "block(ing)" refers to a complete inhibition the biological activity, i.e., no measurable activity.

The terms "protein" and "polypeptide" are used interchangeably herein and refer to any peptide-bond-linked chain of amino acids, regardless of length or post-translational modification. Proteins usable in the present invention (including protein derivatives, protein variants, protein fragments, protein segments, protein epitopes and protein domains) can be further modified by chemical modification. This means such a chemically modified polypeptide comprises other chemical groups than the 20 naturally occurring amino acids. Examples of such other chemical groups include without limitation glycosylated amino acids and phosphorylated amino acids. Chemical modifications of a polypeptide may provide advantageous properties as compared to the parent polypeptide, e.g. one or more of enhanced stability, increased biological half-life, or increased water solubility.

The term "nucleic acid" or "nucleic acid molecule" as used herein is understood as a polymeric or oligomeric macromolecule made from nucleotide monomers. Nucleotide monomers are composed of a nucleobase, a five-carbon sugar (such as but not limited to ribose or 2'-deoxyribose), and one to three phosphate groups. Nucleic acid molecules comprise DNA, RNA, mixtures thereof such as e.g. RNA-DNA hybrids, single-stranded, double-stranded, or more highly aggregated hybridization motifs, and any chemical modifications thereof. Modifications include, but are not limited to, those providing chemical groups that incorporate additional charge, polarizability, hydrogen bonding, electrostatic interaction, and fluxionality to the nucleic acid ligand bases or to the nucleic acid ligand as a whole. Such modifications include, but are not limited to, peptide nucleic acids (PNAs), phosphodiester group modifications (e.g., phosphorothioates, methylphosphonates), 2'-position sugar modifications, 5-position pyrimidine modifications, 7-position purine modifications, 8-position purine modifications, 9-position purine modifications, modifications at exocyclic amines, substitution of 4-thiouridine, substitution of 5-bromo or 5-iodo-uracil; backbone modifications, methylations, unusual base-pairing combinations such as the isobases, isocytidine and isoguanidine and the like. Nucleic acids can also include non-natural bases, such as, for example, nitroindole. Modifications can also include 3' and 5' modifications such as capping with a BHQ, a fluorophore or another moiety. The nucleic acids can e.g. be synthesized chemically, e.g. in accordance with the phosphotriester method (see, for example, Uhlmann, E. & Peyman, A. (1990) Chemical Reviews, 90, 543-584).

As used herein, the term "variant" is to be understood as a polynucleotide which differs in comparison to the polynucleotide or protein from which it is derived by one or more changes in its length or sequence. The polypeptide or polynucleotide from which a nucleic acid variant is derived is also known as the parent polypeptide or polynucleotide. The term "variant" comprises "fragments" or "derivatives" of the parent molecule. Typically, "fragments" are smaller in length or size than the parent molecule, whilst "derivatives" exhibit one or more differences in their sequence in comparison to the parent molecule. Also encompassed are modified molecules such as but not limited to post-translationally modified proteins (e.g. glycosylated, biotinylated, phosphorylated, ubiquitinated, palmitoylated, or proteolytically cleaved proteins) and modified nucleic acids such as methylated DNA. Also mixtures of different molecules such as but not limited to RNA-DNA hybrids, are encompassed by the term "variant". Typically, a variant is constructed artificially, whilst the parent polypeptide or polynucleotide is a wild-type protein or polynucleotide. However, naturally occurring variants are also understood to be encompassed by the term "variant" as used herein. Further, the variants usable in the present invention may also be derived from homologs, orthologs, or paralogs of the parent molecule or from artificially constructed variant, provided that the variant exhibits at least one biological activity of the parent molecule, i.e. is functionally active.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While the invention is illustrated and described in detail in the drawings and description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Any reference signs in the claims should not be construed as limiting the scope.

Throughout this specification and the claims, unless the context requires otherwise, the word "comprise" or "include", and variations such as "comprises/includes" and "comprising/including" are to be understood to imply the inclusion of an element, stated integer, step or a group thereof but not the exclusion of any other element, stated integer, step or a group thereof. The terms "include" and "comprise" are used synonymously.

As used in this specification and the appended claims, the singular forms "a", "an", and "the" include plural referents, unless the content clearly dictates otherwise.

A single unit may fulfill the functions of several features recited in the claims.

The term "essentially", "about" or "approximately" and the like when used in connection with an attribute or numerical value particularly defines exactly the attribute or value and additionally encompass, unless the context indicates otherwise, numerical values within a range having a lower limit that is 0-10% smaller than the indicated numerical value and having an upper limit that is 0-10% larger than the indicated numerical value. The term "about" or "approximately" means preferably ±10%, more preferably ±5%, again more preferably ±3% or most preferably ±0% (referring to the given numeric value, respectively). In each of the invention embodiments, "about" can be deleted. All ranges of values disclosed herein, should refer to and include any and all values falling within said range including the values defining the range.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those skilled in the art that the techniques disclosed in the examples which follow represent techniques used in the present invention to function well in the practice of the invention, and thus can be considered to constitute preferred modes for its practice. However, those skilled in the art should appreciate, in light of the present disclosure that many changes can be made in the specific embodiments which are disclosed and still obtain a like or similar result without departing from the spirit and scope of the invention. Several documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### BRIEF DESCRIPTION OF FIGURES

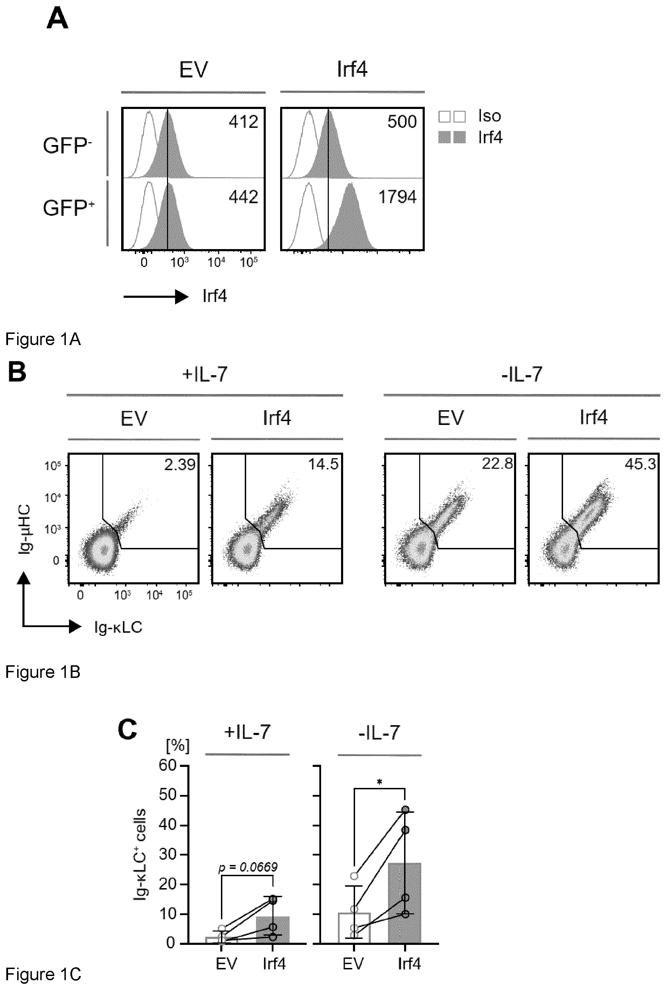

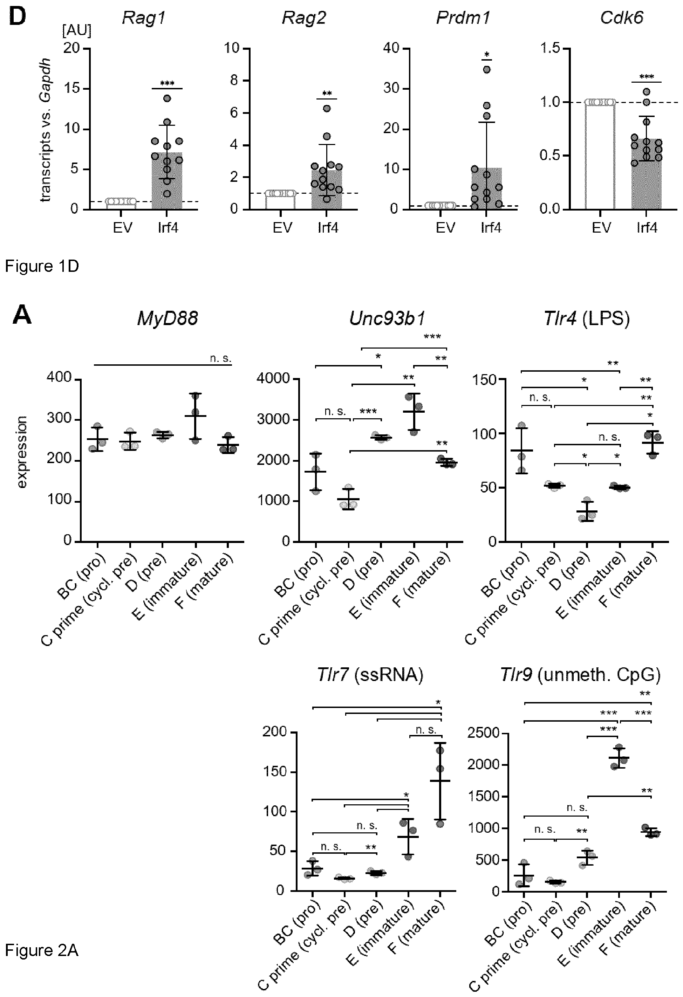

**Figure 1****:** Irf4 overexpression induces Prdm1 in early B cells. A: Ectopic overexpression of Irf4 via retroviral transduction in SLP-65-deficient pre-B cells (February cells). Verification of Irf4 overexpression upon retroviral transduction. SLP-65-deficient pre-B cells were retrovirally transduced with an expression vector encoding Irf4 or the empty vector (EV), respectively. Irf4 expression was determined 3 days post transduction by flow cytometry. Shown data are representative of two individual cell lines. B: Representative flow cytometric analysis of differentiation in SLP-65-deficient pre-B cells (December cells) detected by Ig-kappa LC gene recombination upon ectopic overexpression of Irf4. SLP-65-deficient pre-B cells were retrovirally transduced with expression vectors encoding the transcription factor Irf4 or the empty vector (EV) respectively. 3 days post transduction transduced cells were subjected to IL-7 withdrawal and Ig-µHC and Ig-kappa LC expression were determined by flow cytometry on day 6 post transduction (day 3 upon IL-7 withdrawal). Shown data are representative of 4 individual cell lines (74, February, October, December). C: Quantification of Ig-kappa LC gene positive cells treated as described in (B). Single dots indicate the percentages of Ig-kappa LC+ cells in 4 individual SLP-65-deficient pre-B cell cultures (74, December, February and October) in the respective conditions (n = 4) mean ±SD. Statistical significance was calculated by using the paired t test. D: Rag1 (n = 8), Rag2 (n = 8), Prdm1 (n = 8) and Cdk6 (n = 8) transcripts measured at 3 days post transduction by qRT-PCR. Cells were treated as described in Figure 1B. Irf4- and EV-transduced SLP-65-deficient B cells cultured in +IL-7 medium were FACS-purified. Symbols indicate the expression in cells from individual mice, mean ±SD. Statistical significance was calculated by using the paired one-sample t test (Rag1, Prdm1 and Cdk6) or the Wilcoxon signed rank test (Rag2).

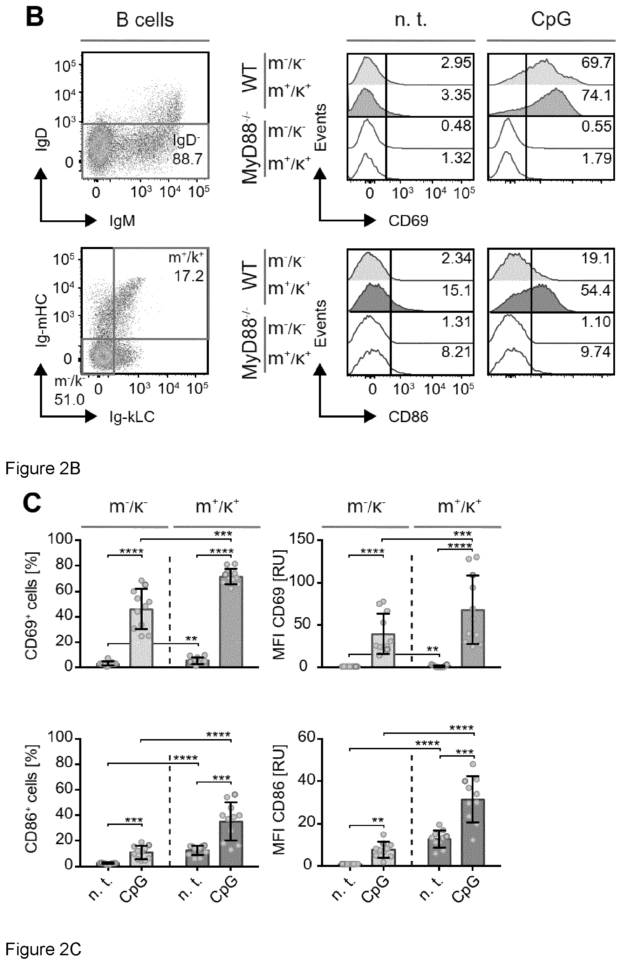

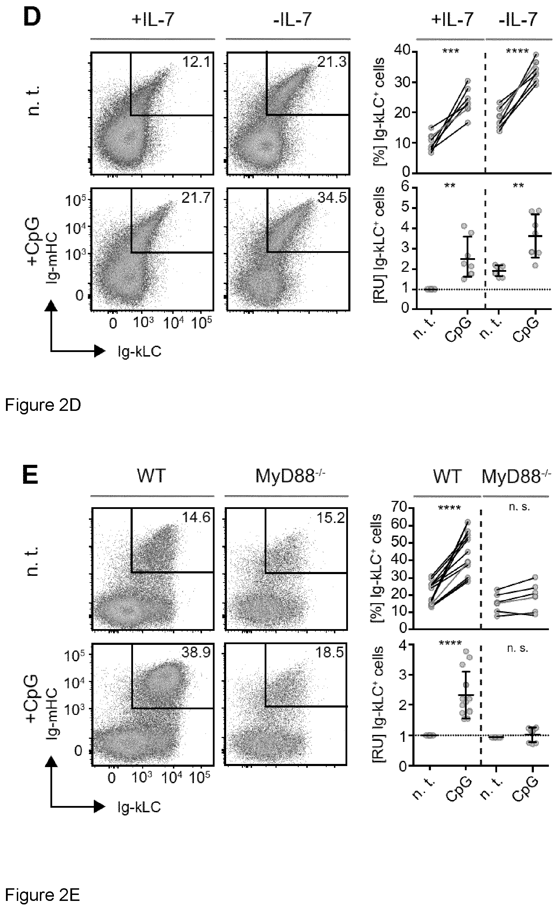

**Figure 2****:** CpG treatment induces differentiation in early B cells. **A:** Analysis of published microarray data on gene expression of MyD88, Unc93b1 and toll like receptors (TLRs) 4, 7 and 9 in Hardy fractions B/C (pro-B cells), C prime (large cycling pre-B cells), D (small pre-B cells), E (newly formed B cells), F (mature recirculating B cells) (n = 3 ± SD). Statistical significance was calculated by applying two-tailed unpaired t-tests. **B:** Bone marrow from wild-type (WT) and MyD88-deficient (MyD88-/- ; MyD88f/f x mb1-cre) mice was cultured in the presence of IL-7 for 3-5 days. The cells were then either left untreated (n. t.) or stimulated with CpG-ODN 1826 (1 µM) for further 2 days. Cells were pre-gated on viable CD19+/IgD- cells and expression of activation markers CD69 and CD86 was compared in IgM+ (lg-µ+/k+) and IgM- cells (lg-µ-/k-) cells. Numbers in the histogram plots indicate the percentage of positive cells, representative of 11 independent experiments. **C:** Quantification of percentages (left) and mean fluorescence intensities (MFI) (right, MFI as relative units (RU), normalized to the respective MFI of non-treated IgM-/IgD- (Ig-µ-/kappa-) B cells shown in Fig. B. Single dots represent independent experiments from individual mice, dots in red indicate data shown in the histograms. Numbers on top of the bars state the fold change between the mean values of untreated and CpG-treated cells (n = 11, mean ± SD). Statistical significance was calculated by applying a 2-way ANOVA. **D:** Bone marrow from WT and MyD88-deficient mice was cultured in IL-7-supplemented medium for 3-5 days. Subsequently the cells were either left untreated (n. t.) or stimulated with CpG-ODN 1826 (1 µM) for 2 days and cultured in presence or absence of IL-7. Cells were pre-gated on viable CD19+/IgD- cells and expression of Ig-µHC and -kappa LC was assessed by flow cytometry (FACS). Numbers in the dot plots (left) indicate the percentages of lg-µ+/kappa+ cells. Diagrams (right) depict the percentages of Ig-µ+/kappa+ cells (top) and the fold change of lg-µ+/kappa+ cells (bottom, normalized to expression levels measured in untreated cells cultured in presence of IL-7) in the respective conditions. Single dots represent independent experiments from individual mice; red dots indicate data shown in the dot plots (n = 7, mean ±SD). significance was calculated by applying a 2-way ANOVA. E: Bone marrow from WT and MyD88 was treated as described in Fig. 2B and expression of Ig-µHC and -kappa LC was assessed by flow cytometry. Numbers in the dot plots (left) indicate the percentages of lg-µ+/kappa+ cells. Diagrams (right) depict the percentages of Ig-µ+/kappa+ cells (top) and the fold change of lg-µ+/kappa+ cells (bottom, normalized to expression levels measured in untreated cells cultured in presence of IL-7) in the respective conditions. Single dots represent independent experiments from individual mice, dots in red indicate data shown in the histograms (WT n = 13, 607 MyD88-/- n = 6, mean ±SD). Statistical significance was calculated by applying a 2-way ANOVA.

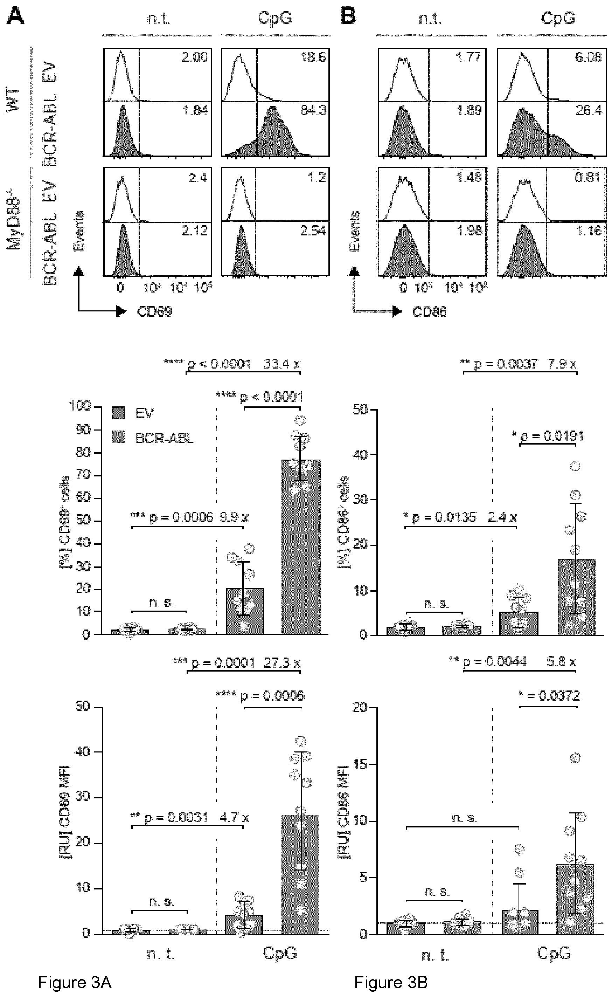

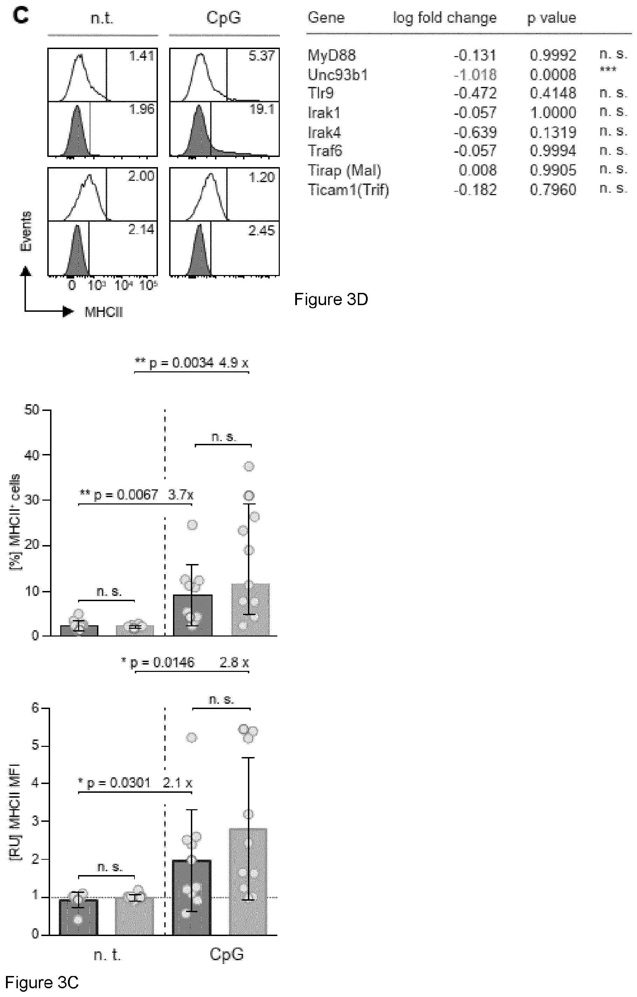

**Figure 3****:** BCR-ABL1 transformed B cells are more sensitive to TLR9 stimulation. WT and MyD88-deficient bone marrow cells were cultured in the presence of IL-7 for 3 - 6 weeks until stably growing IL-7-dependent cell lines were established. Cells were then either retrovirally transduced with a BCR-ABL-encoding expression vector (BCR-ABL) or the empty vector (EV). The resulting cell lines cells were then either left untreated (n. t.) or stimulated with CpG-ODN 1826 (2 µM) for 3 days. Cells were pre-gated on viable CD19+/IgM-/IgD-/GFP+ cells and expression of activation markers CD69 (A), CD86 (B) and MHC class II (C) was compared in BCR-ABL transformed and non-transformed cells. Numbers in the histogram plots indicate the percentages of positive cells, representative of 10 independent experiments. Bar diagrams below compare percentages (top) and the MFI (bottom, MFI as relative units, normalized to the MFI of untreated cells) of CD69 and CD86 B cells shown in the histogram plots. Single dots represent independent experiments from individual mice, red dots indicate data shown in the histograms. Numbers on top of the bars state the fold change between the mean values of untreated and CpG-treated cells (n = 10, mean ±SD). Statistical significance was calculated by applying paired t-tests. **D:** Expression of the indicated genes was compared between EV and BCR-ABL1-transduced cells by deep sequencing. Significant differences in fold changes of gene expression are highlighted in red (reduction) or green (increase).

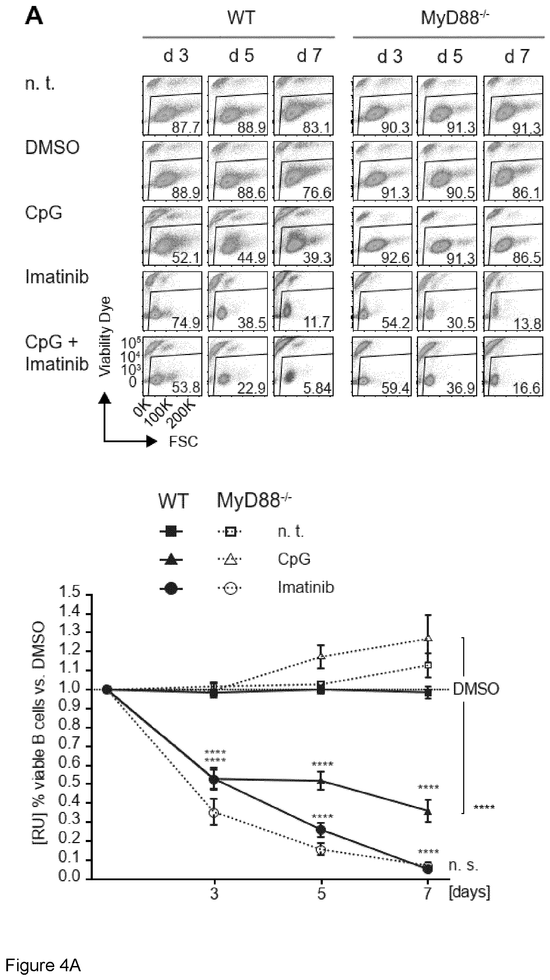

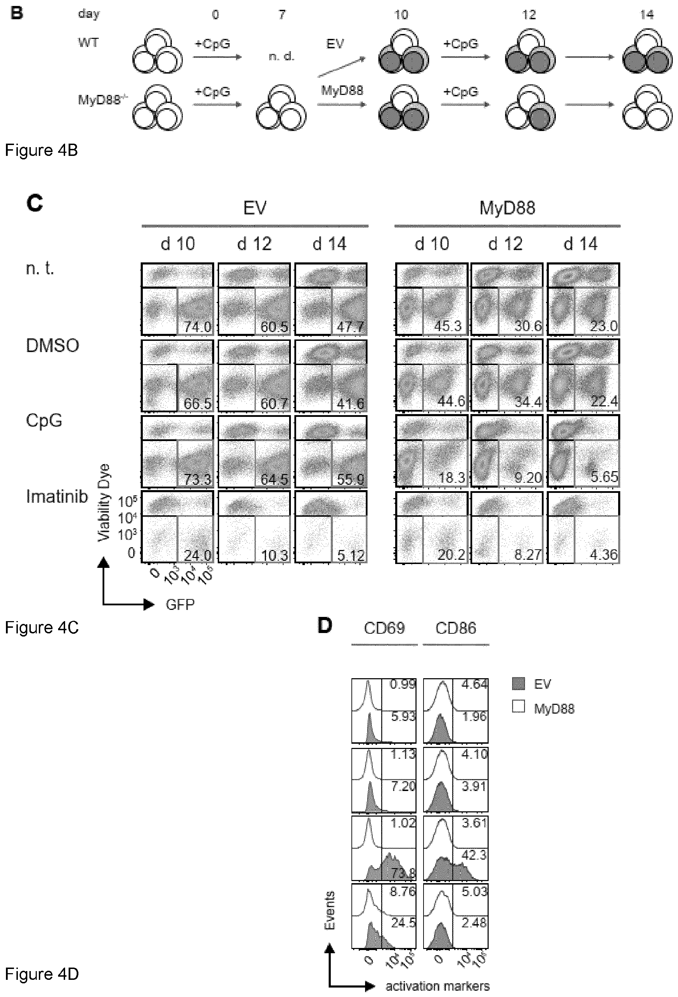

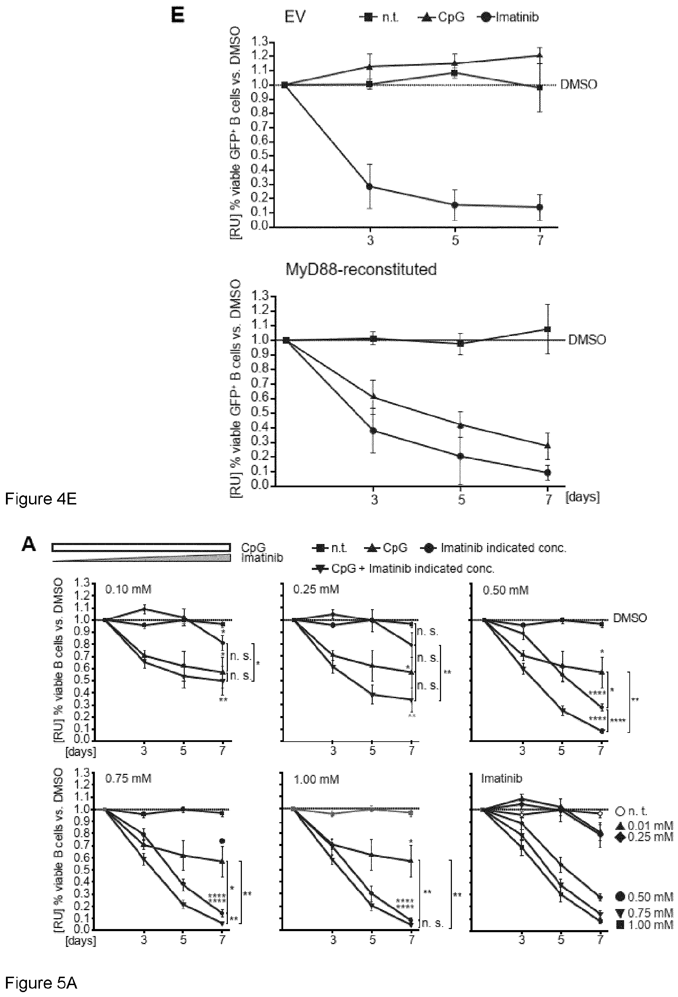

**Figure 4****:** CpG treatment leads to activation and loss of BCR-ABL1 transformed B cells. BCR-ABL-transformed WT and MyD88-deficient bone marrow cells were either left untreated (n. t.), stimulated with DMSO, CpG-ODN 1826 (2 µM + DMSO) or the tyrosine-kinase inhibitor Imatinib (1 µM in DMSO) for 7 days. **A:** Representative FACS data of viable cells in the respective conditions on days 3, 5 and 7 following treatment. Numbers in the dot plots indicate the percentage of viable cells (left). Right: Quantified percentages of viable CD19+ untreated cells (n. t.) and CpG- (blue) or Imatinib (red) - treated cells normalized on the percentages measured in DMSO-treated cells on indicated time points after treatment. Shown data are representative of up to 15 technical replicates in up to 2 cell lines (n) established from an individual mouse (WT filled squares and solid lines, n = 15, 9 individual mice; MyD88-/- open squares and dashed lines, n = 14, 10 individual mice; mean ±SEM). Statistical significance was calculated by applying two-tailed unpaired t-tests. Blue and red stars indicate significance of difference between the treatment condition indicated by the color and the untreated control at the indicated time points. **B:** Schematic overview of experimental procedure. BCR-ABL transformed WT and MyD88-deficient precursor B cells were stimulated as described in Fig. 4A. After 7 days of treatment, CpG-resistant MyD88-deficient B cells were washed and reconstituted either with an expression vector encoding MyD88 or the respective EV by retroviral transduction. After transduction the cells were again either left untreated or stimulated with 2 µM CpG-ODN #1826. The survival of GFP+ cells was monitored for further 7 days. n. d. = not detectable. **C:** Representative flow cytometric analysis of EV- or MyD88 reconstituted BCR-ABL-transformed either left untreated (n. t.), stimulated with DMSO, CpG-ODN 1826 (2 µM + DMSO) or the tyrosine-kinase inhibitor Imatinib (1 µM in DMSO) for 7 days. **D:** Representative flow cytometric data of CD69 and CD86 expression in GFP+ EV or MyD88-transduced cells from Fig. 4C in the different treatment conditions. **E:** Quantification of viable GFP+ cells from Fig. 4B reconstituted either with EV or MyD88 either left untreated or upon stimulation with CpG (2 µM CpG-ODN 1826 + DMSO) and Imatinib (1 µM). Percentages of viable GFP+ cells were normalized on DMSO-treated cells at the respective time points.

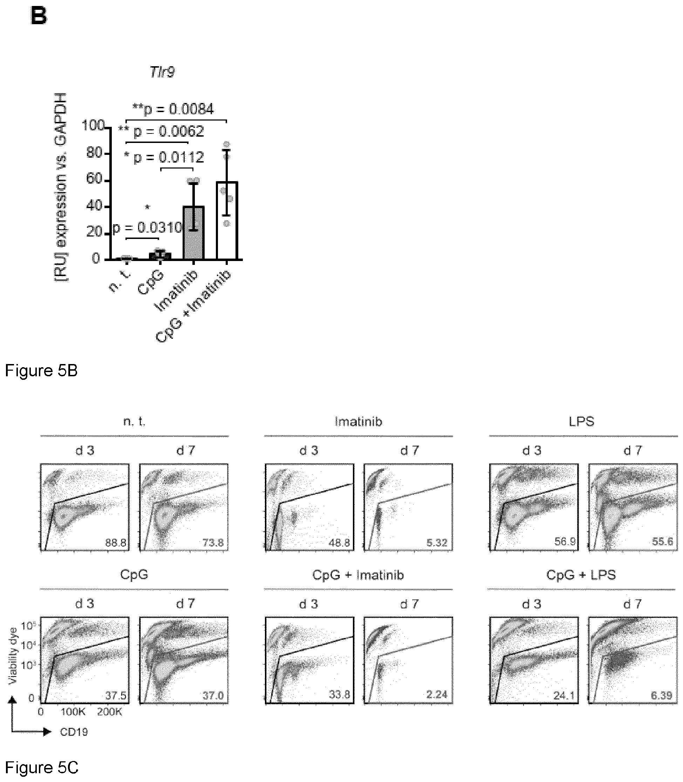

**Figure 5****:** Combination of Imatinib with CpG elicits synergistic effects. **A:** Titration of Imatinib in presence and absence of CpG. The concentration of CpG-ODN 1826 was maintained at a constant concentration of 2 µM, while the Imatinib concentration was increased from 0.1 µM (left) to 1.00 µM in the single treatment and the combination with CpG-ODN 1826. Survival of BCR-ABL-transformed WT cells was monitored and analyzed as described in Fig. 4A, (WT n = 7; mean ±SEM). Statistical significance of differences compared to n. t. was calculated by applying two-tailed unpaired t-tests. Colored stars indicate significance of difference between the treatment condition indicated by the color and the untreated control at the indicated time points. **B:** TLR9 expression levels were analyzed by reverse transcriptase qPCR in BCR-ABL-transformed WT cells in the indicated treatment conditions at 3 days following stimulation (concentrations as in Fig. A) (WT n = 5 cell lines from 5 individual mice; ±SEM). Statistical significance was calculated by applying two-tailed paired t-tests. C: Representative flow cytometry data from BCR-ABL-transformed murine WT BCPs (d) 3 and 7, which were either left untreated (n. t.), stimulated with CpG 1826 (2 µM), LPS (1 µg/ml), the TKI Imatinib (1 µM) or combinations of CpG with LPS or Imatinib, respectively. Numbers in the dot plots indicate the percentage of viable cells. Combination of CpG with LPS or Imatinib elicits synergistic effects Representative flow cytometry data from BCR-ABL-transformed murine WT BCPs (d) 3 and 7, which were either left untreated (n. t.), stimulated with CpG 1826 (2 µM), LPS (1 µg/ml), the TKI Imatinib (1 µM) or combinations of CpG with LPS or Imatinib, respectively. Numbers in the dot plots indicate the percentage of viable cells.

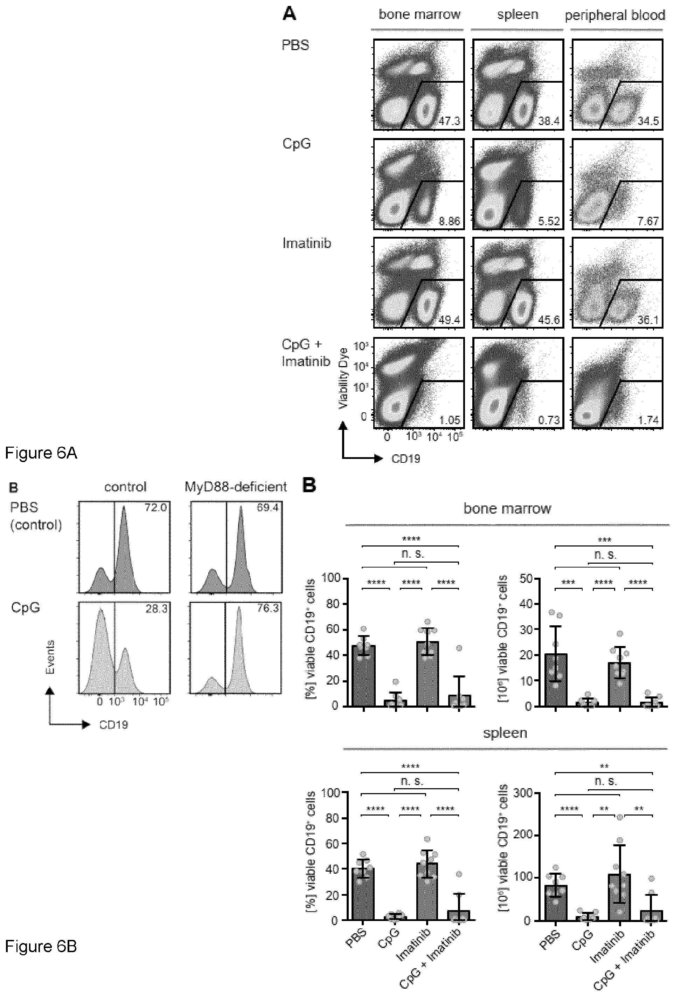

**Figure 6****:** CpG kills BCR-ABL1 transformed B cells in vivo. 0.5-1x10⁶ BCR-ABL transformed precursor B cells of WT origin were adoptively transferred into 8 to 10 weeks old Rag2/γc double-deficient mice by injection into the tail vein. The mice were randomly assigned to treatment groups that obtained CpG-ODN 1826 (4 g/kg body weight), Imatinib (50 g/kg body weight), a combination of these substances, or PBS by intraperitoneal injection (i.p.). The mice were treated every 3 to 4 days (twice per week) with the respective substances and were analyzed approximately 3 weeks after transfer of the cells when the first animal in the cohort showed symptoms of leukemia. **A:** Representative flow cytometric analysis of cells from bone marrow, spleens and peripheral blood of mice from the indicated treatment groups. Numbers in the dot plots represent the percentage of positive cells. **B:** Right: Quantification of viable GFP+/CD19+ precursor B cells (left, percentages; right absolute cell numbers) in bone marrow (top) and spleens (bottom) of mice treated either with PBS (n=8), CpG-ODN 1826 (n=8), Imatinib (n=9) and CpG-ODN 1826 + Imatinib (n=8), mean ±SD. Statistical significance was calculated by applying the unpaired two-tailed t-test. Left: Representative flow cytometric analysis of peripheral blood cells from recipient mice transplanted with either with BCR-ABL-transformed BCPs derived from control or MyD88-deficient mice and treated either with CpG or PBS. The finding that MyD88-deficient cells appear to be resistant to CpG-treatment supports the previous notion that CpG directly causes cell-death of BCR-ABL-transformed BCPs and that potential CpG-mediated functions in myeloid cells, which are still present in Rag2/common g-chain double-deficient mice, are not sufficient for reducing the leukemic cells

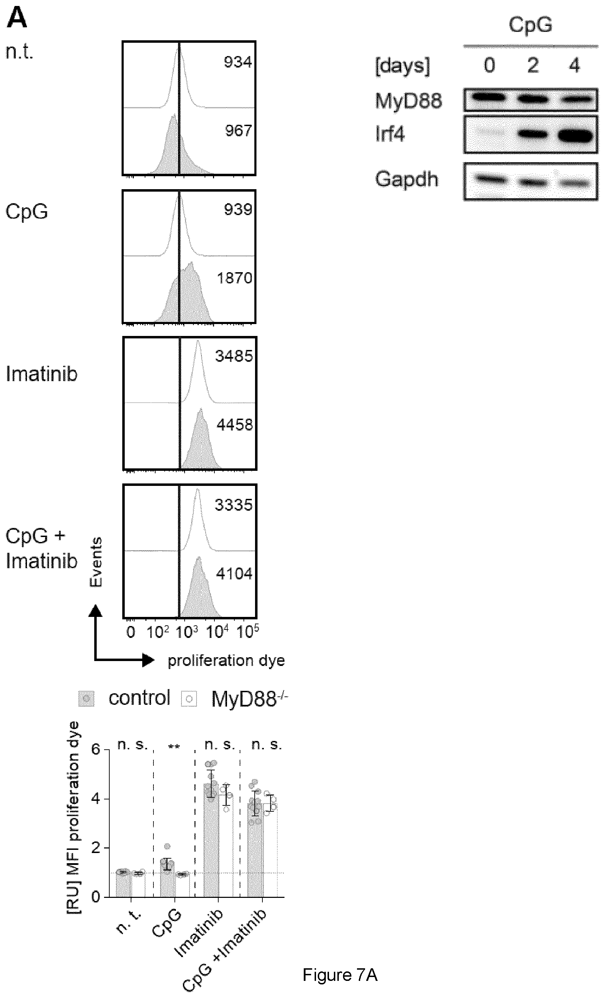

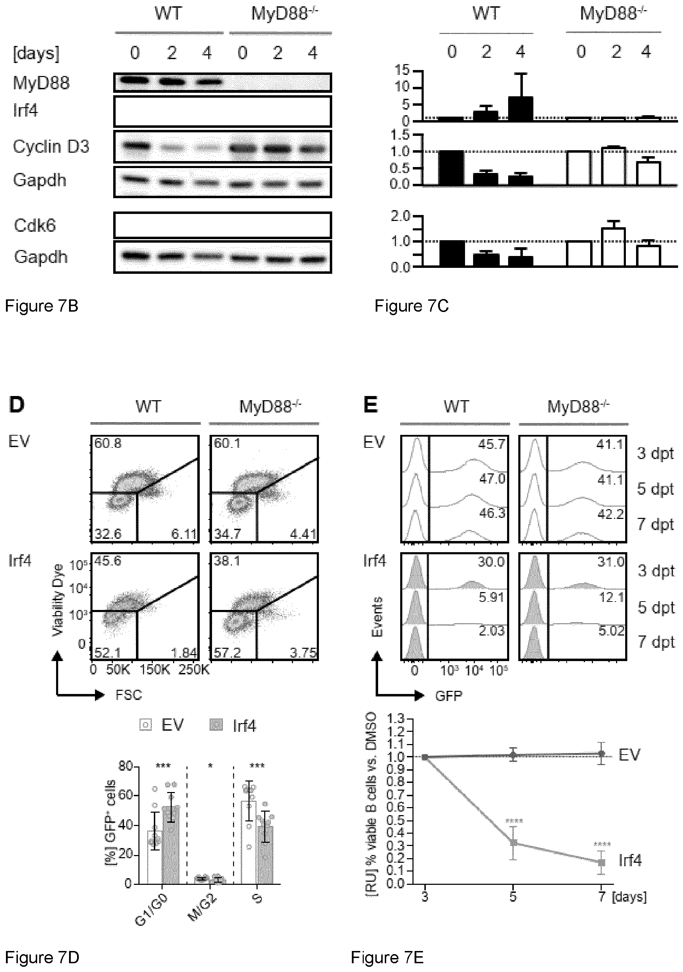

**Figure 7****:** Differentiation blockades in BCR-ABL1-transformed B cells can be overcome by signaling via TLR9. **A:** Left: BCR-ABL-transformed WT and MyD88-deficient bone marrow cells were labeled with proliferation dye and left untreated (n. t.), stimulated with DMSO, CpG-ODN 1826 (2 µM + DMSO), Imatinib (1 µM in DMSO) or a combination of both substances. At day 3 post stimulation the MFIs were compared between cells from WT and MyD88-deficient origin and the among the different treatment groups. Numbers in the representative histograms (top) indicate the MFI. MFIs were quantified (bottom) and normalized to the MFI of DMSO-treated cells. WT: 12 cell lines from 6 individual mice; MyD88-/-: 4 cell lines from 2 individual mice; mean ±SD). Statistical significance was calculated by applying two-tailed unpaired t-tests. Right: Induction of Irf4-expression upon treatment of BCR-ABL-transformed BCPs with CpG Immunoblot analysis of MyD88 and Irf4 in in CpG-treated BCR-ABL-transformed BCP. Cells were harvested and analyzed at 0, 2 and 4 days upon treatment with CpG 1826 (2 µM). GAPDH served as loading control. Following stimulation of BCR-ABL-transformed BCPs with CpG, we observed increased expression of Irf4 , which has been shown to bind to the enhancers of the IgL gene loci to drive transcriptional activation required for IgL gene recombination. **B:** Immunoblot analysis of MyD88 and Irf4 in CpG-treated BCR-ABL-transformed WT and MyD88-deficient bone marrow cells. Cells were harvested and analyzed at 0, 2 and 4 days upon treatment with CpG-ODN 1826 (2 µM). GAPDH served as loading control. Data are representative of at least 2 cell lines from individual mice per genotype. **C:** Immunoblot analysis of Irf4, Cyclin D3, Cdk6 and p-p53 in CpG-treated BCR-ABL-transformed WT and MyD88-deficient bone marrow cells. Cells were treated as shown described in Fig. 7B. Data are representative of at least 2 cell lines from individual mice per genotype. **D:** BCR-ABL transformed WT (n = 6) and MyD88-deficient (n = 4) B cells were subjected to retroviral transduction with Irf4 or the respective EV and the cell cycle was analyzed by flow cytometry at day 3 upon transduction. The percentages of cells in the different cell cycle phases (G0/G1, M/G2 and S) were quantified and statistical significance was calculated by applying the paired two-tailed t-test. **E:** BCR-ABL transformed WT (n = 6) and MyD88-deficient (n = 4) B cells were subjected to retroviral transduction with Irf4 or the respective EV. Quantified percentages of viable GFP+ cells at days 5 and 7 were normalized to the percentages measured at 3 days after transduction, mean ±SD). Statistical significance was calculated by using the two-way ANOVA.

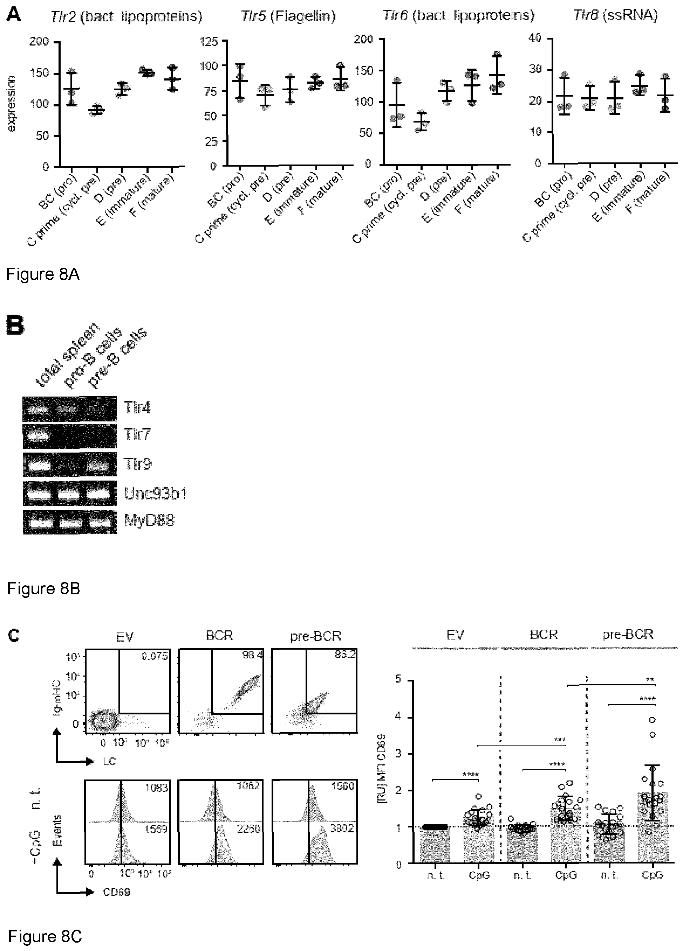

**Figure 8****:** CpG treatment induces activation and differentiation in early B cells. **A:** Expression of Toll-like receptors (TLRs) 2, 5, 6 and 8 in Hardy fractions B/C (pro-B cells), C prime (cycling pre-B cells) D (pre-B cells), E (newly formed B cells) and F (recirculating B cells) (n = 3 ± SD). Statistical significance was calculated by using the unpaired t-test. **B:** Expression of TLRs 4, 7, Unc93b1 and MyD88 was compared in IL-7-dependent bone marrow-derived pro- and pre-B cell lines by RT-PCR. cDNA from total splenocytes served as positive control. **C:** Rag2/λ5-deficient pro-B cells were reconstituted either with a combination of heavy (HC) and conventional light chain (LC) of the B1-8 BCR (BCR), the B1-8 HC and λ5 surrogate LC to form the pre-BCR, or the empty vector (EV). BCR surface expression was verified by flow cytometry (left top panel). Numbers in the dot plots indicate the percentage of cells in the positive gate. Cells were then either left untreated or stimulated for 2 days with 0.1 µM CpG-ODN and the activation state was assessed by measurement of CD69 (left bottom panel). Numbers in the histograms indicate the mean fluorescence intensity (MFI). The MFI of CD69 was quantified and normalized to the MFI measured in non-treated EV-transduced cells (n = 19, mean ± SD). Statistical significance was calculated by using either the paired or the unpaired t-test.

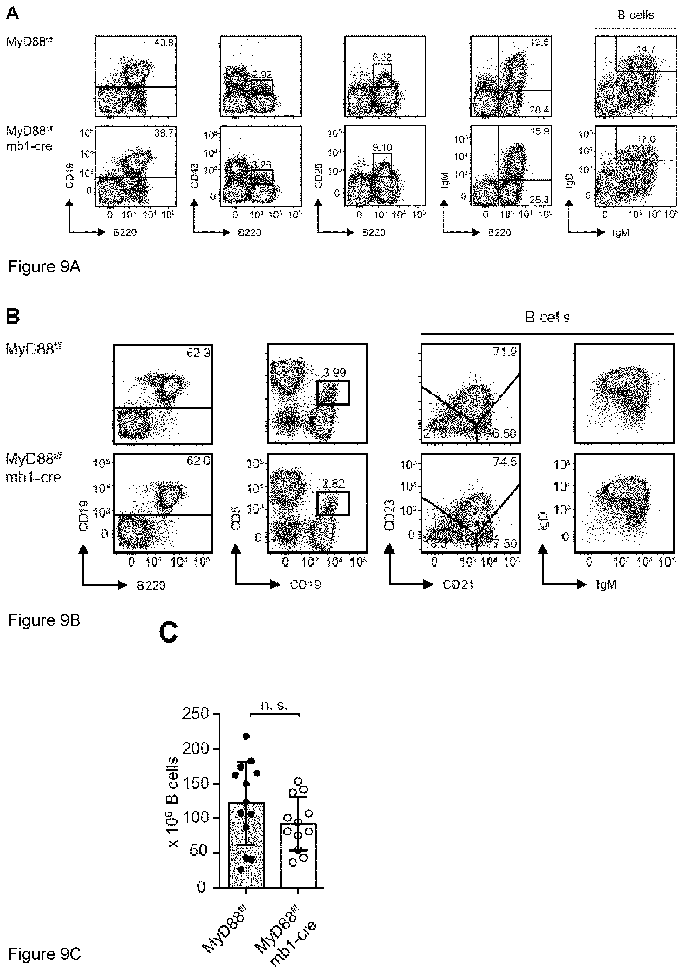

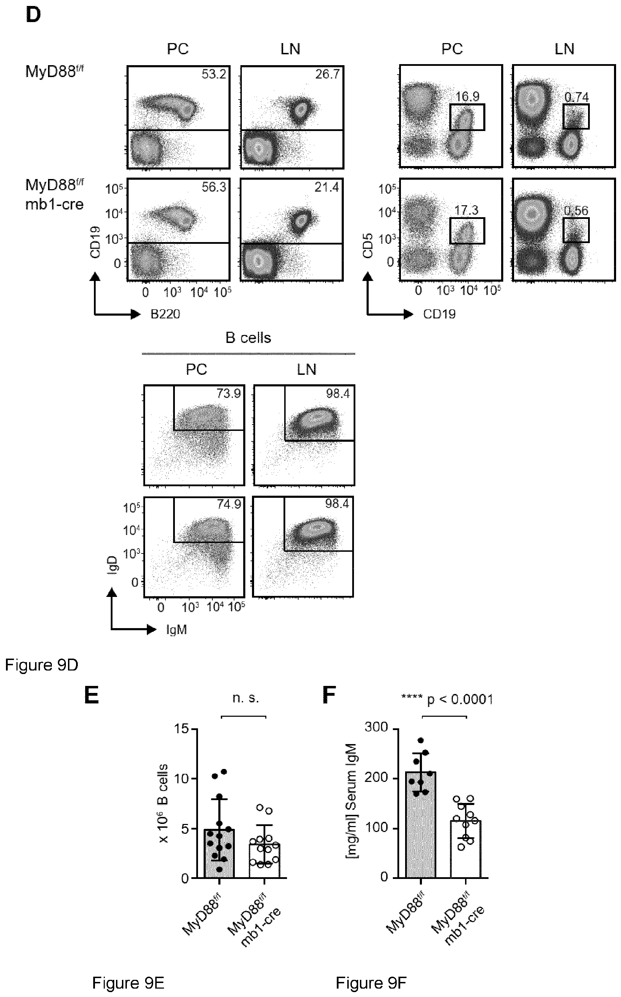

**Figure 9****:** MyD88-deficiency does not affect B cell development. A, B, **D:** MyD88f/f and MyD88f/f x mb1-cre mice were sacrificed at the age of 8 weeks. Cells isolated from bone marrow (A), spleens (B), peritoneal cavities (PC) and lymph nodes (LN) (D) were stained for the indicated markers and analyzed by flow cytometry. Shown dot plots are representative of 13 MyD88f/f and 12 MyD88f/f x mb1-cre mice. C, **E:** Quantification of B cells in spleens (C) and bone marrow (E) of MyD88f/f and MyD88f/f x mb1-cre mice. MyD88f/**F:** n = 13; MyD88f/f x mb1-cr**E:** n = 12; mean ± SD. Statistical significance was calculated by using the unpaired t-test. **F:** IgM concentrations measured in the sera from MyD88f/f and MyD88f/f x mb1-cre mice. MyD88f/**F:** n = 8; MyD88f/f x mb1-cr**E:** n = 10; mean ± SD. Statistical significance was calculated by using the unpaired t-test.

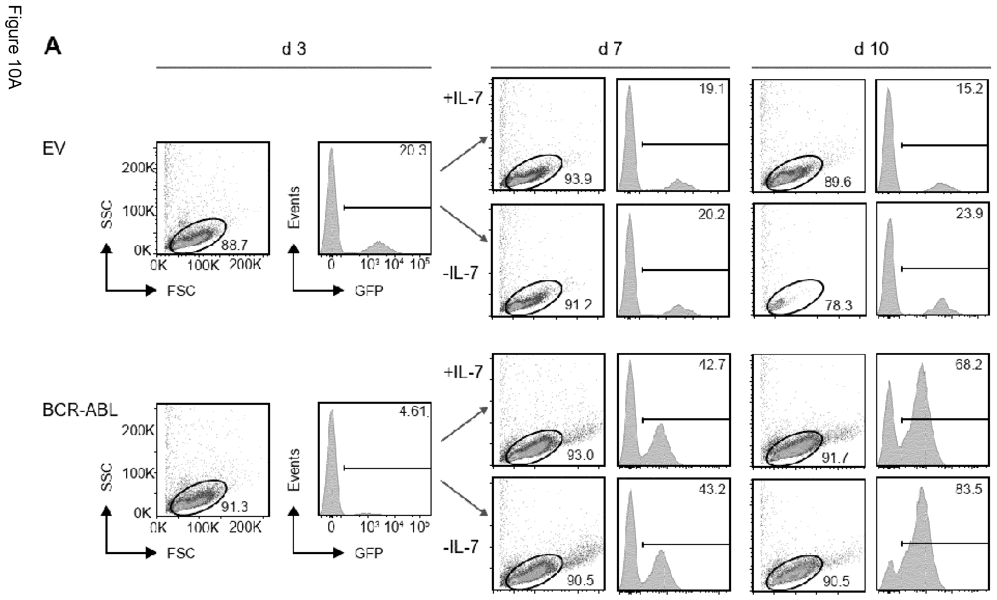

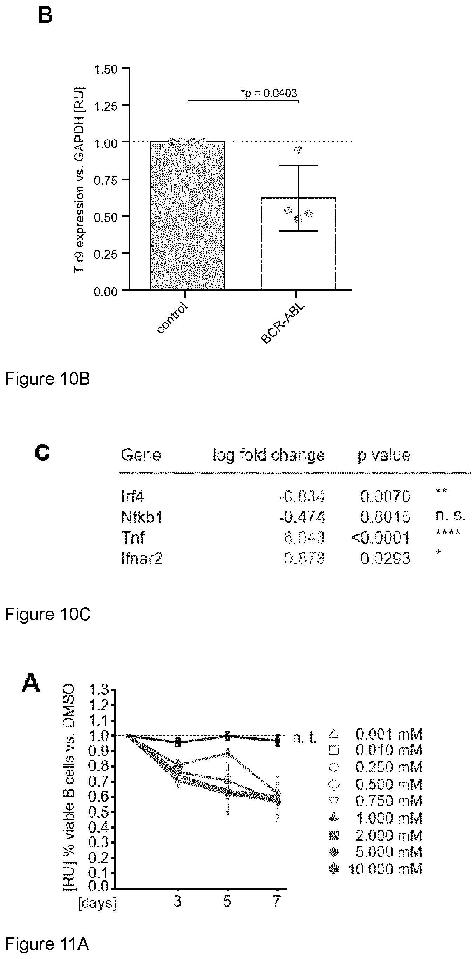

**Figure 10****:** BCR-ABL transformed cells are more sensitive to TLR stimulation. **A:** Bone marrow-derived wildtype cells were subjected to retroviral transduction with expression vectors encoding either BCR-ABL (lower panel) or the respective empty vector (EV) (upper panel). The transduction efficiency was determined 3 days later. After additional two days (d5) the culture was split and while some cells were further cultured in IL-7 supplemented medium (upper and third row), IL-7 was slowly withdrawn from the other half of the culture (second and bottom row). Cells were pre-gated on viable cells. The percentage of GFP+ cells was measured again at day 7 and 10.**B**: RNA was extracted from non-transformed control cells and their BCR-ABL-transformed counterparts and TLR9 expression levels were determined by TaqMan-based qRT-PCR run in triplicates 4 cell lines were used from 2 individual mice. Statistical significance was calculated by using the paired t-test. **C:** Expression of the indicated genes was compared between EV and BCR-ABL-transduced cells by deep sequencing. Significant differences in fold changes of gene expression are highlighted in red (reduction) or green (increase).

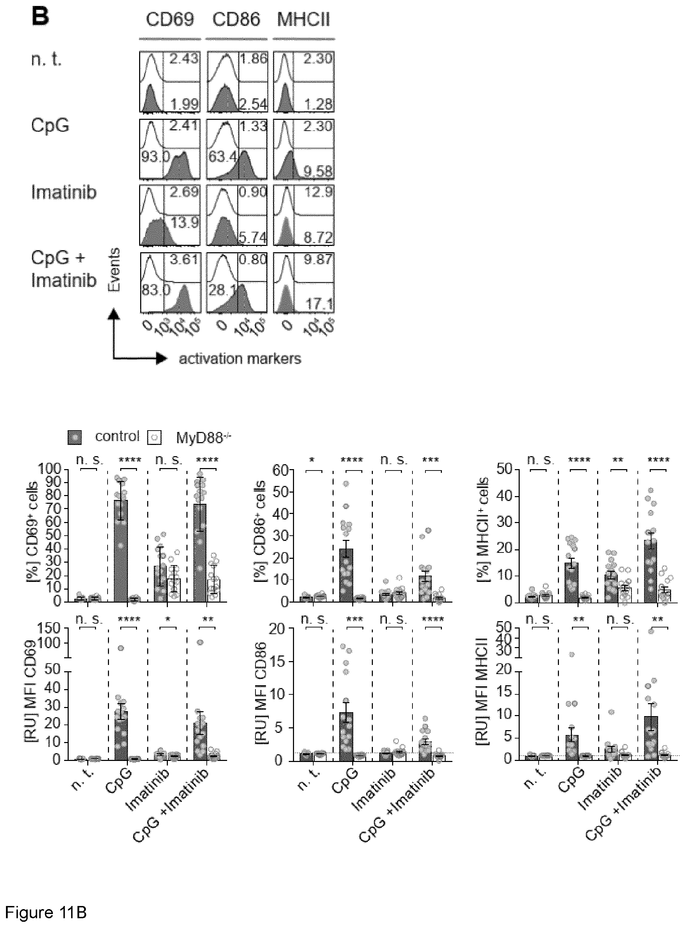

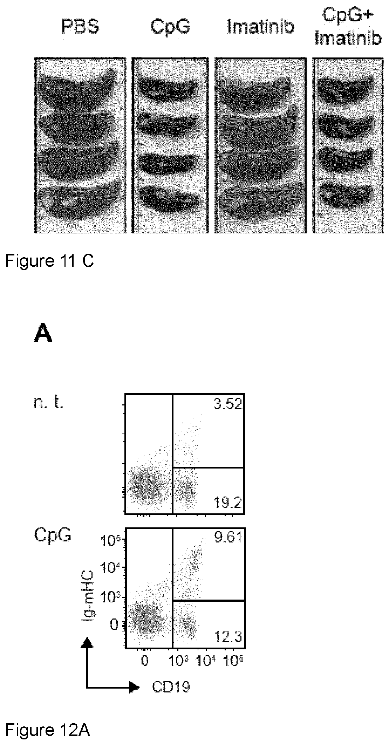

**Figure 11****:** CpG activates and kills BCR-ABL transformed B cells (related to Fig. 4). **A:** Titration of CpG-ODN 1826 from 0.001 µM (left) to 10.00 µM. Survival of BCR-ABL-transformed WT cells was monitored and analyzed as described in Fig. 4A, (WT n = 7; mean ±SEM). Statistical significance of differences compared to n. t. was calculated by applying two-tailed unpaired t-tests. Colored stars indicate significance of difference between the treatment condition indicated by the color and the untreated control at the indicated time points. **B:** BCR-ABL-transformed WT and MyD88-deficient bone marrow cells were either left untreated (n.t.), stimulated with DMSO, CpG-ODN 1826 (2 µM + DMSO) or the tyrosine-kinase inhibitor Imatinib (1 µM in DMSO) for 7 days. Activation markers CD69, CD86 and MHC class II were measured in viable CD19+ BCR-ABL transformed cells in the indicated treatment conditions on day 3. Numbers in the histogram plots indicate the percentages of positive cells. Bar diagrams (right) compare percentages (top) and the MFI (bottom, MFI as relative units, normalized to the MFI of DMSO-treated cells) of the indicated activation markers in BCR-ABL transformed cells shown in the histogram plots. Shown data are representative of up to 15 technical replicates in up to 2 cell lines (n) established from an individual mouse (WT n = 15, 9 individual mice; MyD88-/- n = 14, 10 individual mice; mean ±SEM). Statistical significance of differences compared to n. t. was calculated by applying two-tailed unpaired t-tests. **C:** CpG prevents expansion of human TKI-resistant BCP-ALL cells in vivo Macroscopic appearance of spleens from recipient mice transplanted with patient-derived TKI-resistant BCP-ALL cells and treated either with CpG 1826 (4 g/kg body weight) or PBS by intraperitoneal injection (i.p.). 1 x 10⁶ cells were adoptively transferred into 8 to 10 weeks old NSG mice by injection into the tail vein. The mice were treated 5 times per week with the respective substances and were analyzed as soon as the first animal showed symptoms of leukemia. CpG treatment limited the expansion of TKI-resistant Ph+ BCP-ALL cells in vivo, as shown by the considerably reduced spleen size
**Figure 12****:** Human Ph+ pre-B ALL cells are sensitive to CpG treatment. **A:** CD34+ precursor B cells from human cord blood were cultured for 28 days in vitro. Cells were either left untreated or stimulated for 10 days with 0.1µM CpG-ODN 2006 and analyzed by flow cytometry for Ig-µHC surface expression. Shown data are representative of at least 2 independent experiments with cells from individual donors.

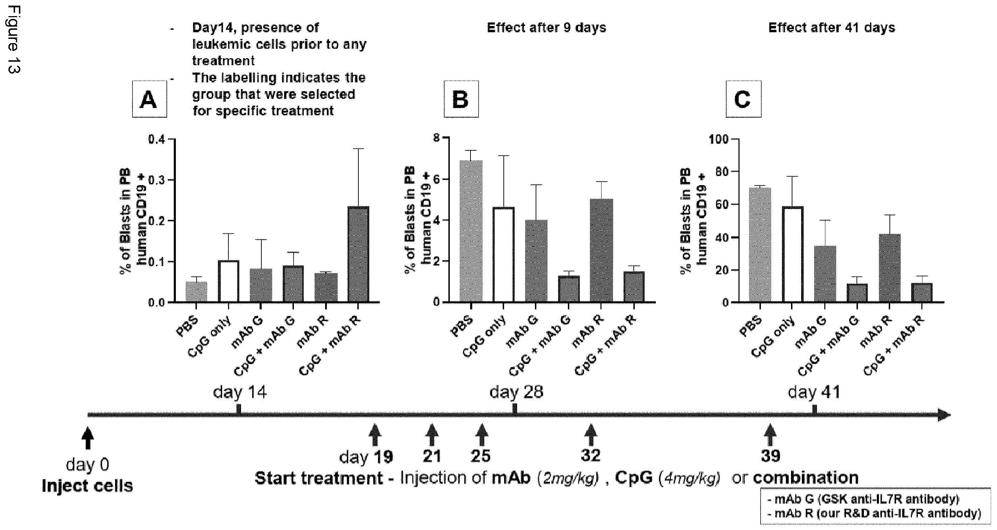

**Figure 13****:** In vivo effect of injecting monoclonal antibodies against anti-IL7R in blocking pro/pre-B cell leukemia development after injecting the leukemia cells. **A:** Day 14, presence of leukemic cells prior to any treatment. **B:** Effect after 9 days of treatment, day 28. **C:** Effect after 41 days.
**Figure **9:** CpG prevents expansion of human TKI-resistant BCP-ALL cells in vivo Macroscopic appearance of spleens from recipient mice transplanted with patient-derived TKI-resistant BCP-ALL cells and treated either with CpG 1826 (4 g/kg body weight) or PBS by intraperitoneal injection (i.p.). 10⁶ cells were adoptively transferred into 8 to 10 weeks old NSG mice by injection into the tail vein. The mice were treated 5 times per week with the respective substances and were analyzed as soon as the first animal showed symptoms of leukemia.

To show that treatment with TLR ligands may represent an alternative strategy for therapeutic intervention in TKI-resistant human Ph+ BCP-ALL, NSG mice were transplanted with TKI-resistant human samples. Transfer experiments were performed with patient-derived BCP-ALL cells harboring the BCR-ABL T315I gatekeeper mutation which renders the cells resistant to Imatinib-treatment. CpG treatment even limited the expansion of TKI-resistant Ph+ BCP-ALL cells in vivo, as shown by the considerably reduced spleen size.

### EXAMPLES

### Example 1 - Material and Methods

### Mice

MyD88f/f mice (Hou et al., 2011) were purchased from The Jackson Laboratory and crossed to mb1-cre (CD79a-cre) (Hobeika et al., 2006) for B cell-specific pre-mediated MyD88 ablation. 0.5-1 x 10⁶ BCR-ABL1 transformed B cells were adoptively transferred into immunodeficient Rag2/common g chain-double deficient mice (Colucci et al., 1999) via tail vein injection. The mice were randomly assigned to treatment groups that obtained either CpG-ODN 1826 (4 g/kg body weight), Imatinib (50 g/kg body weight), a combination of both substances, or PBS by intraperitoneal injection (i.p.). The mice were treated every 3 to 4 days (twice per week) and were analyzed approximately 3 weeks after transfer of the cells when the first animal in the cohort showed symptoms of disease. These experiments were performed in compliance with animal research permit #1322 at the regional board of Tübingen, Germany. All mice were bred and housed in the animal facility of Ulm University under specific pathogen free conditions. All animal experiments were done in compliance with the guidelines of the German law and were approved by the Animal Care and Use Committees of Ulm University and the local government.

### Cell culture and biochemistry

Phoenix cells were cultured in Iscove's medium (Biochrom AG) containing 5% heat-inactivated FCS (Sigma-Aldrich), 2 mM L-glutamine (Gibco), 100 U/ml penicillin (Gibco), 100 U/ml streptomycin (Gibco), and 50 µM 2-mercaptoethanol. Cell lines from control, MyD88-deficient and Rag2/I5-deficient mice (Meixlsperger et al., 2007; Storch et al., 2007) were established by culturing bone marrow from the respective mice in medium supplemented with the supernatant of J558L plasmacytoma cells stably transfected with a vector encoding murine IL-7 (Abdelrasoul et al., 2018). Human Ph+ BCP-ALL cell lines Sup-B15 (Quentmeier et al., 2011) and TOM-1 (Okabe et al., 1987), were cultured in McCoy's Medium 5A (biowest) and RPMI (Gibco), respectively. Both media were supplemented with 20% heat-inactivated FCS (Sigma-Aldrich), 2 mM L-glutamine (Gibco), 100 U/ml penicillin (Gibco), 100 U/ml streptomycin (Gibco), 0.01 M HEPES buffer (Gibco) and 50 µM 2-mercaptoethanol (Gibco). Cells of murine origin were treated with CpG-ODN 1826, human cell lines with CpG-ODN 2006 (both from Apara Bioscience). CpG and Imatinib (Selleckchem) were used in concentrations, stated in the figure legends.

### Plasmids and retroviral transduction

Viral supernatants were generated using the Phoenix retroviral producer cell line as described in the manufacturer's instructions. Phoenix cells were cultured in Iscove's culture medium +5% FCS and transfected by using GeneJuice (Merck Millipore). Supernatants, containing retroviral particles, were harvested after 48 h. For transduction the respective cells were mixed with supernatants and centrifuged at 300 g at 37°C for 3 h. Transduced cells were returned to culture for at least 3 days and analyzed by flow cytometry. pMIG-BCR-ABL1 was kindly provided by W.S. Pear (University of Pennsylvania, Philadelphia, PA). The plasmid for expression of IRF4 was described previously (Abdelrasoul et al., 2018). Full-length cDNA encoding MyD88, was subcloned into the retroviral vector backbone pMI in order to generate the MyD88-IRES-GFP expression vector.

### Flow cytometry

For flow cytometric analysis, cell suspensions were pretreated with α-CD16/CD32 Fc-Block (2,4G2; BD Biosciences) and stained by standard procedures. Dead cells were excluded by staining with Fixable Viability Dye eFluor 780 (eBioscience). Cells were stained by using the following antibodies:

| **Antigen** | **Conjugate** | **Clone** | **Company** |
|---|---|---|---|
| CD5 | eFluor 450 or PE | 53-7.3 | eBioscience |
| CD19 | PerCP-Cy5.5 | 1D3 BD | Biosciences |
| CD19 | eFluor 450 or APC | eBio1D3 | eBioscience |
| CD21/CD35 | APC or PE-Cy7 | 7E9 | BioLegend |
| CD23 | PE or Biotin | B3B4 BD | Biosciences |
| CD25 | APC | PC61 BD | Biosciences |
| CD43 | FITC | S7 BD | Biosciences |
| CD45R (B220) | PE-Cy7 or PerCP-eFluor 710 | RA3-642 | eBioscience |
| CD69 | APC | H1.2F3 | eBioscience |
| CD86 | PE-Cy7 | GL-1 | BioLegend |
| CD138 | PE | DL-101 | eBioscience |
| CD138 | Brilliant Violet 421 | 281-2 | BioLegend |
| MHCII | eFluor 450 | M5/114.15.2 | eBioscience |
| IgD | FITC, PE or Biotin | 11-26 | SouthernBiotech |
| IgD | APC | 11-26 | eBioscience |
| IgM | FITC | polyclonal | SouthernBiotech |
| IgM | eFluor 450 | eB121-15-F9 | eBioscience |
| IgM | Cy5 | polyclonal | Jackson Immunoresearch |
| Kappa | PE or Biotin | polyclonal | SouthernBiotech |

Biotin was detected by using Streptavidin Qdot 605 (Molecular Probes - Invitrogen). Cells were acquired at a FACSCanto II flow cytometer (BD Biosciences). If not indicated otherwise, numbers in the dot plots indicate the percentages of cells in the respective gate, numbers in the histograms indicate the mean fluorescence intensity (MFI).

### Cell cycle analysis

Cells were pulsed for 30 min with bromodeoxyuridine BrdU (APC BrdU Flow Kit, BD Biosciences) and stained according to instructions given by the manufacturer.

### Proliferation assay

Cells were labeled with eFluor 670 Proliferation Dye (Invitrogen) according to manufacturer's instructions. Cells were incubated with CpG for 3 days before analysis at the flow cytometer.

### Immunoblotting

Concentration-adjusted amounts of denatured protein lysates were separated by SDS-gel electrophoresis on a 10 % gel and transferred onto a nitrocellulose membrane. The membrane was blocked for 1 h with PBS-T (PBS/0.05% Tween (Sigma)) containing 5% Bovine Serum Albumin (BSA, Serva) and washed before probing with the primary antibody diluted in blocking solution overnight.

| **Antigen** | **Origin** | **Clone** | **Company** |
|---|---|---|---|
| Irf4 | rabbit | | Abcam |
| CDK6 | mouse | DCS83 | Cell Signaling |
| Cyclin D3 | mouse | DCS22 | Cell Signaling |
| MyD88 | rabbit | Cell | Signaling |
| GAPDH | rabbit | D16H11 | Cell Signaling |

After washing steps, the membrane was incubated for 1 h at RT with horseradish peroxidase (HRP)-coupled secondary goat anti-rabbit antibodies, diluted in PBS-T/1% BSA. Antibodies in excess were washed off and stained proteins were detected with ECL Ultra Solution kit (Lumigen) on a Fusion SL advanced imaging system (Vilber Lourmat).

### Enzyme-linked immunoblot assay

96-well plates (NUNC, maxisorp) were coated with polyclonal a-mouse IgM antibody (SouthernBiotech) and blocked with buffer containing 1% BSA. Dilutions of a-mouse IgM (SouthernBiotech) were used as standard. The concentration of IgM was determined by detection with Alkaline-phosphatase-labeled a-mouse IgM (Southern Biotech). P-Nitrophenylphosphate (Genaxxon) in diethylamine buffer was added and data were acquired at 405 nm using a Multiskan FC ELISA plate reader (Thermo Scientific).

### Quantitative PCR

RNA was extracted by using the ReliaPrep RNA Cell Miniprep System (Promega) and cDNA synthesis was performed with the RevertAid Reverse Transcriptase kit (Thermo Fisher) as indicated by the manufacturer. qPCR analysis was performed in triplicates using the TaqMan-Probe mixes Mm00446193_m1 (TLR9) and Mm99999915_g1 (GAPDH) (Applied Biosystems) with TaqMan gene expression mastermix (Applied Biosystems). qPCR data were acquired on a StepOnePlus Real-Time thermocycler (Applied Biosystems) and analyzed with the StepOne Software v2.3. Results were calculated by applying the DCT-method.

Statistical analysis: Graphs were created, and statistical analysis was performed by using GraphPad Prism (Version 6.0h) software. The numbers of individual replicates or mice (n) is stated in the figure legends or the figure itself. If not indicated otherwise p-values were calculated by applying two-tailed unpaired t-tests. P values <0.05 were considered to be statistically significant (n. s. = not significant; * p≤0.05; ** p≤0.01; *** p≤0.001; **** p≤0.0001). Example 2 - Toll-like receptors are expressed and functional in early B cells

### Example 2 - Toll-like receptors are expressed and functional in early B cells

To investigate if early B cells potentially have the capacity to sense PAMPs, the inventors analyzed published microarray data on gene expression levels of TLRs during B cell development (Bagger et al., 2016; Heng et al., 2008; Painter et al., 2011). Transcript levels of TLRs such as 2, 6, 7 and 9 seem to increase with the developmental progress from large (cycling) pre-B cells via the small pre-B cell stage to immature B cells (Fig. 2A and Fig. 8A). Interestingly, in particular the expression of TLR9 appears to be drastically elevated at early stages of B cell development as compared with other members of the TLR family (Fig. 2A and Fig. 8A-B). Moreover, transcripts of central signaling molecules such as MyD88 or Unc93b1 required for TLR9 function are detectable at abundant amounts, indicating that the TLR signaling machinery might be functional in early B cells. To test this, the inventors stimulated bone marrow cells from control and MyD88f/f x mb1-cre mice (in which the common TLR signaling adaptor MyD88 had been conditionally inactivated by mb1-cre, hereafter referred to as "MyD88-/-"), with CpG-oligodeoxynucleotides (CpG, CpG-ODN). Early B cells responded readily to CpG-treatment by upregulation of activation markers CD69 and CD86 (Fig. 2B). Strikingly, upon TLR9 stimulation, immature B cells (IgM+/IgD+, Ig-µ+/k+) expressed significantly higher levels of CD69 and CD86 compared to pro-/pre-B cells (IgM- cells, Ig-µ-/k-). To assess whether the presence of a BCR induces TLR expression, the inventors reconstituted Rag2/l5 double deficient (DKO) pro-B cells, lacking endogenous BCR expression, with the B1-8 Ig-µHC and either the I5-LC to generate cells expressing the autonomously active pre-BCR or the conventional Ig-ILC to obtain the classical B1-8 BCR (Fig. 8C). Exposure of reconstituted Rag2/I5-DKO to CpG confirmed the finding that cells equipped with a BCR show stronger activation, as measured by CD69 induction, compared to pro-B cells. Notably, already pro-B cells showed a capacity to respond to TLR9 stimulation. Together, these findings suggest that already early B cells can sense unmethylated CpG motifs by TLR9 and that the sensitivity of those cells is increased in the course of B cell development via BCR expression.

Since TLR signaling has effects on B cell differentiation, the inventors investigated whether CpG treatment promotes Ig-kLC gene recombination in pre-B cells lacking the signaling adaptor SLP-65 (Herzog et al., 2008). Indeed, stimulation with CpG resulted in elevated levels of Ig-kLC B cells both in presence and absence of IL-7 (Fig. 2D). Comparable results were obtained when early B cells of wild-type (WT) origin were exposed to CpG, whereas MyD88-deficient B cells failed to induce Ig-kLC gene recombination under these conditions (Fig. 2E). These findings indicate that the TLR9 signaling machinery is expressed and functional already in early B cells and contributes to B cell differentiation and maturation. Thereby these results point toward a potential role of MyD88 during early B cell development.

To address this the inventors characterized B cell development in MyD88f/f x mb1-cre mice (Fig. 9). Conversely, the inventors could not observe significant changes neither in the absolute B cell numbers in bone marrow (Fig. 9C) and spleens (Fig. 9E), nor in the composition of the individual B cell compartments found in bone marrow (Fig. 9A), spleens (Fig. 9B), lymph nodes and peritoneal cavities (Fig. 9D). However, the inventors detected less IgM in the sera of MyD88f/f x mb1-cre mice, as compared to MyD88f/f mice (Fig. 9E).

### Example 3 - BCR-ABL1 transformed cells show increased sensitivity to TLR stimulation

As the inventors' results indicated a potential contribution of TLR9 signaling to B cell differentiation, it was hypothesized that this may play a role in overcoming differentiation blockades, as they occur in transformed B cell progenitors (Bennett et al., 1976; Lowenberg et al., 1999). To test this hypothesis, the inventors generated IL-7 dependent pre-B cell lines from bone marrow cells of MyD88-deficient and control mice and retrovirally transduced them in vitro either with an expression vector encoding the oncogenic fusion protein BCR-ABL1 or the respective empty vector (EV) (Fig. 10A) (Duy et al., 2010). The cells are murine cells from WT or derived from MyD88 KO mice. Cells transduced with BCR-ABL1 accumulated rapidly and survived after IL-7 withdrawal in contrast to EV-transduced cells (Fig. 10A) (Appelmann et al., 2015; Daley and Baltimore, 1988; Jena et al., 2002; Sillaber et al., 2000). Since the capacity to be activated by CpG appears to be attenuated in non-transformed (EV) pre-B cells during the in vitro selection process of several weeks that leads to the generation of a stably growing IL-7-dependent cell line (Fig. 2 and 3), the inventors first investigated whether the ability to sense unmethylated CpG motifs is maintained following transformation with BCR-ABL1. Surprisingly, the responsiveness to CpG was restored upon transformation with BCR-ABL1 as the majority of cells upregulated CD69 (Fig. 3A), while smaller fractions showed also CD86 (Fig. 3B) and MHCII (Fig. 3C). Comparable results were obtained when primary early B cells were transformed with BCR-ABL1 without previously being cultured to generate a stable IL-7-dependent cell line (data not shown). Thus, BCR-ABL1-transformed B cells which are blocked in differentiation and do not express BCR, show strong activation upon CpG-exposure, which is highly reminiscent of the elevated CD69 levels reached only in primary BCR-expressing B cells (Fig. 2B).

In order to understand how BCR-ABL1-transformation results in this enhanced predisposition to activation upon TLR9 stimulation, the inventors tested whether BCR-ABL1 induces TLR9 expression thus increasing the sensitivity for CpG. Comparison of expression levels between EV- and BCR-ABL1-transduced B cells revealed neither a significant upregulation of TLR9 expression nor of any other down-stream component associated with TLR9 signaling (Fig. 3D). Analysis by qRT-PCR to verify the deep sequencing data revealed that TLR9 is actually down-regulated in -ABL1-transformed cells (Fig. 10B).

Together, these data suggest that BCR-ABL1-expression confers sensitivity to TLR9 stimulation possibly by altering TLR signaling components however not by upregulating TLR9 expression as such.

### Example 4 - CpG treatment leads to activation and loss of BCR-ABL1 transformed B cells

While stimulating BCR-ABL1-transformed B cells with CpG, the inventors observed that a considerable number of cells was lost under these conditions (Fig. 4A-B). Titration of CpG in BCR-ABL1-transformed cell lines showed that cell survival was affected in a dose-independent manner (Fig. 11A). As shown, BCR-ABL1-transformed B cell lines from control mice responded vigorously to CpG by upregulating the activation markers CD69, CD86 and MHCII, while in MyD88-deficient BCR-ABL1-transformed B cells no signs of activation were detectable. Notably, treatment of BCR-ABL1-transformed B cells with Imatinib also resulted in mild activation, independent from MyD88 (Fig. 11B). While both, BCR-ABL1-transformed cell lines from control and MyD88-deficient mice showed comparable sensitivity to treatment with the tyrosine kinase inhibitor (TKI) Imatinib, MyD88-deficient BCR-ABL1-transformed B cells were selectively resistant to CpG treatment (Fig. 4A). In fact, 15 of 19 BCR-ABL1-transformed B cell lines, generated from the bone marrow of control mice, were sensitive to CpG treatment, whereas 9 of 9 cell lines originating from MyD88-deficient mice showed resistance, indicating, that this phenomenon is common to BCR-ABL1-transformed B cells with an intact TLR9 signaling pathway. When MyD88 was reconstituted in MyD88-deficient BCR-ABL1-transformed B cells by retroviral transduction (Fig. 4B-C), cells that were previously unaffected by CpG-treatment upregulated activation markers CD69 and CD86 (Fig. 4D) and the majority of MyD88-reconstituted cells was lost within 7 days of CpG treatment (Fig. 4E). These findings indicate that CpG treatment leads to cell death in BCR-ABL1-transformed B cells in a MyD88-dependent manner.

### Example 5 - Combination of Imatinib with CpG elicits synergistic effects

Stimulation with both CpG and Imatinib, a small molecule inhibitor of tyrosine kinases, was most efficient in limiting survival of BCR-ABL1-transformed B cells (Fig. 4A), demonstrating that the combination of both substances elicits synergistic effects. To examine this in more detail, the inventors titrated Imatinib in presence and absence of a constant concentration of CpG (Fig. 5A). Low concentrations of Imatinib by itself showed very little effect on the survival of BCR-ABL1-transformed B cells, while the average effect of exposure to 2 µM CpG was comparable to treatment with 0.25 to 0.5 µM Imatinib. Interestingly, addition of CpG to low concentrations of Imatinib (ranging from 0.1 to 0.75 µM), that barely affected survival, significantly increased the loss of BCR-ABL1-transformed B cells (Fig. 5A).

As the inventors detected elevated levels of transcripts for TLR9 following treatment with Imatinib (Fig. 5B), it is conceivable that Imatinib treatment renders BCR-ABL1-transformed B cells more sensitive to CpG treatment. Thus, the intrinsic capacity of BCR-ABL1-transformed B cells to sense unmethylated CpG motifs may possibly provide a new therapeutic approach for treatment of Ph+ BCP-ALL.

### Example 6 - Human Ph+ BCP-ALL cells are sensitive to CpG treatment

To evaluate whether CpG treatment could be applicable in humans, the inventors tested CpG treatment in human BCR-ABL1-positive BCP-ALL cell lines Sup-B15 (Quentmeier et al., 2011) and TOM-1 (Okabe et al., 1987).

Human Ph+ BCP-ALL cells of human were either left untreated, stimulated with DMSO, CpG-ODN 2006 (10 µM + DMSO), Imatinib (2 µM in DMSO) or a combination of both. FACS data of viable Sup-B15 and TOM-1 cells under the respective treatment conditions determined on days 2, 4 and 8 following beginning of stimulation were collected. Quantified percentages of viable CD19+ untreated cells, CpG- or Imatinib-treated cells and cells that were exposed to a combination of both compounds were measured and normalized on the percentages measured in DMSO-treated cells at certain time points after treatment.

Survival of Sup-B15 cells was affected by CpG treatment. In human cell lines, the combination of Imatinib and CpG was also most efficient in limiting survival of BCP-ALL cells. Although Sup-B15 cells are reported to be resistant to treatment with Imatinib (Quentmeier et al., 2011), Imatinib showed a synergistic effect when combined with CpG. These data indicate that BCR-ABL1-transformed BCP-ALL cells of human origin are also responsive to stimulation via TLR9 and concomitant exposure to Imatinib and CpG sensitizes the cells and thus may improve treatment efficiency.

### Example 7 - CpG reduces the number of BCR-ABL1-transformed precursor-B cells in vivo

Although Imatinib treatment is efficient in vitro, it was reported to show only marginal effects in vivo. To test if CpG is effective and possibly enhances Imatinib treatment in the context of an entire organism, the inventors adoptively transferred murine BCR-ABL1-transformed B cells into immunodeficient Rag2/common gamma chain-double deficient mice. The mice were randomly distributed to treatment groups that obtained CpG, Imatinib, a combination of either substances, or PBS by intraperitoneal injection (i.p.) every 3 to 4 days. The treatment with CpG was tolerated well as the animals showed normal behavior and maintained their body weight. The mice were analyzed as soon as the first animal in the group showed symptoms of disease (approximately after 3 weeks after transfer of the cells). Detailed analysis by flow cytometry revealed that already CpG-treatment alone significantly restricted the expansion of BCR-ABL1-transformed B cells in bone marrow, spleen and peripheral blood of all mice analyzed (Fig. 6A). Treatment with Imatinib however led to no reduction of the burden by BCR-ABL1-transformed B cells as compared with the PBS-treated control group. Although no significant difference could be detected in the overall percentages and absolute cell numbers among the Imatinib and PBS groups, some individuals that had obtained Imatinib, were found to exhibit higher percentages of BCR-ABL1-transformed B cells in the spleens that also translated into elevated absolute numbers, as compared to PBS-controls. There is no significant statistical difference among the group that received CpG and the group that was treated with the combination of CpG and Imatinib, as CpG-treatment by itself was already highly efficient in the removal of BCR-ABL1-transformed B cells. CpG treatment resulted in very low percentages of remaining leukemic cells and thus small residual populations of the respective cells could still be detected (Fig. 6A). Treatment with the combination of both substances led to elimination of this residual cell population in 3 of 9 animals. Thus, also in the group receiving the combination of both substances some individual animals were found with elevated numbers of BCR-ABL1-transformed B cells as compared with mice in the group that received CpG alone (Fig. 6B).

Taken together, the inventor's findings suggest that CpG-ODNs by themselves or by boosting the effects of a tyrosine kinase inhibitor may represent a powerful novel approach in the treatment of Ph+ ALL. Moreover, the observation that the animals with the highest burden of leukemic cells were found in the group in which Imatinib was administered implies that low concentrations may even have adverse effects.

### Example 8 - Differentiation blockades in BCR-ABL1-transformed B cells can be overcome by signaling via TLR9

In order to characterize the underlying molecular mechanism of CpG-induced cell death, the inventors investigated whether CpG-treatment inhibits proliferation of BCR-ABL1-transformed B cells. While treatment with Imatinib considerably affected proliferation of both, control and MyD88-deficient BCR-ABL1-transformed B cells, stimulation with CpG only had minor effects on cell division of BCR-ABL1-transformed B cells from control mice (Fig. 7A). Moreover, the inventors could not observe a synergistic effect upon combination of both substances, indicating, that CpG inhibits proliferation, but this is unlikely to be the reason for the observed loss of cells. As non-transformed early B cells responded to CpG-treatment with Ig-k gene recombination and this is usually accompanied by a loss of those cells in vitro (Herzog et al., 2008), the inventors hypothesized that a similar mechanism might drive CpG-induced loss of cells also in BCR-ABL1-transformed B cells. Surface expression of Ig-kappa LC was not detectable in CpG-treated BCR-ABL1-transformed B cells (data not shown) but interestingly the inventors observed increased expression of interferon regulatory factor 4 (Irf4) (Fig. 7B), a transcription factor which has been shown to be essential for Ig-kappa gene recombination (Herzog et al., 2009; Johnson et al., 2008; Ma et al., 2006; Muljo and Schlissel, 2003). Irf4 upregulation following CpG treatment occurred specifically in control but not in MyD88-deficient BCR-ABL1-transformed B cell lines. A more detailed analysis revealed that in control BCR-ABL1-transformed B cells, CpG-treatment led to reduced amounts of Cyclin D3 and CDK6 which have been shown to form a complex that contributes to the transition from G1 to the S phase of the cell cycle (Malumbres, 2014) (Fig. 7C). Moreover, the inventors also detected phosphorylation and thus activation of the tumor suppressor p53 (Ashcroft et al., 1999; Dai and Gu, 2010) (Fig. 7C). Interestingly, already ectopic overexpression of Irf4 by retroviral transduction was sufficient to impair cell cycle progression in BCR-ABL1-transformed B cell lines of control and MyD88-deficient origin to comparable extent, resulting in significantly decreased amounts of cells in the M and S-phase (Fig. 7C). Moreover, the majority of BCR-ABL1-transformed B cells transduced with Irf4 were lost within a week, a time frame in which the inventors also observed the loss of cells upon treatment with CpG (Fig. 7D), suggesting that exposure to CpG induces expression of differentiation-promoting factor Irf4 which is sufficient impair the cell cycle and lead to a loss of BCR-ABL1-transformed B cells.

Taken together, the inventor's data indicate that the capacity of sensing PAMPs via TLRs is maintained or may be even enhanced in BCR-ABL1-transformed B cell progenitors and that this intrinsic ability may represent a novel therapeutic approach for the treatment of Ph+ ALL.

### Example 9 - Antibodies against anti-IL7R

To study the efficacy of monoclonal antibodies against anti-IL7R in blocking pro/pre-B cell leukemia development, the inventors tested the effect of an anti-IL7R antibody injection 2-3 weeks after injecting leukemia cells, such as murine BCR-ABL1-transformed B cells and confirming their presence in the peripheral blood of recipient mice.

Under these conditions, with established Ph+ ALL, anti-IL7R treatment alone inhibited leukemia development and rescued mice. CpG injection, when applied at the same time as leukemic cells (day 0 or 1), even prevented leukemia development.

Further, the inventors tested whether a combined treatment of anti-IL7R antibodies with CpG (CpG-ODN 1826) could block leukemic cell proliferation. The inventors injected patient-derived primary human leukemia cells, which are Philadelphia chromosome-positive and imatinib-resistant, into immunodeficient mice (intraperitoneal injections, NSG mice or Rag1/common gamma C) and monitored leukemia cell appearance in the blood at day 14 post-injection. Injected phosphate-buffered saline (PBS) served as control and as basis for the CpG and antibody injections. As expected, leukemic cells were observed in all injected mice (Fig. 13A).

The inventors selected groups of three mice for injections with the indicated combinations, using mice with increased leukemic cell numbers in blood for combined antibody/CpG treatment. The inventors used two different antibodies: "mAb G" (anti-IL7R antibody from GlaxoSmithKline) and "mAb R" (anti-IL7R antibody from R&D, sequenced and patented for leukemia treatment). The treatment started on day 19 with the indicated combinations. By day 28, mice treated with "mAb G + CpG" or "mAb R + CpG" showed a clear reduction in leukemic cells in the blood compared to control mice treated with PBS (Fig. 13B). Single treatments with either anti-IL7R antibody (mAb G or mAb R) or CpG alone also had an inhibiting effect on leukemic cell expansion in this setting but were less effective.

On day 41, the experiment had to be stopped because the control mice treated with PBS showed around 70% leukemic cells in the blood and this the sign (marker or limit) for termination of the experiment (Fig 13C). However, the combined treatment of CpG and an anti-IL7R antibody (mAb G or mAb R) resulted in a clear reduction of the leukemic cells in the blood of recipient mice suggesting that this combined treatment might be a highly effective way to block the expansion of manifested leukemia.

## Claims

1. A composition comprising a Toll-like receptor agonist and an Interleukin-7 (IL-7) antagonist.

2. A kit comprising a Toll-like receptor agonist and an Interleukin-7 (IL-7) antagonist.

3. The composition of claim 1 or the kit of claim 2, wherein the Toll-like receptor is selected from the group consisting of TLR2, TLR4, TLR5, TLR6, TLR7, and TLR9; wherein preferably the Toll-like receptor is selected from the group consisting of TLR2, TLR6, TLR7 and TLR9; wherein more preferably the Toll-like receptor is TLR9.

4. The composition or kit of anyone of the preceding claims, wherein the Toll-like receptor agonist is a CpG oligonucleotide comprising at least one preferably at least two unmethylated CpG dinucleotides (CpG motifs), preferably the CpG is a DNA molecule containing at least one preferably at least two unmethylated CpG dinucleotides.

5. The composition or kit of anyone of the preceding claims, wherein the CpG oligonucleotide comprises or consists of a class A, class B or class C CpG oligonucleotide, preferably the CpG oligonucleotide comprises or consists of a class B CpG oligonucleotide.

6. The composition or kit of anyone of the preceding claims, wherein all or at least one of the internucleotide linkages of the CpG oligonucleotide are phosphorothioate linkages.

7. The composition or kit of anyone of the preceding claims, wherein the CpG oligonucleotide comprises or consists of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684; or the CpG oligonucleotide comprises or preferably consists of a variant of an oligonucleotide sequence selected from the group consisting of SEQ ID NO: 1-11, CpG-ODN 1826, ODN1018, K3-CpG, CPG T1, T2, T3, ODNBW006, ODN D-SL01, ODN2006, and ODN684, wherein in the variant 1 to 4 nucleotide(s) is/are substituted, added or deleted, and the variant comprises at least one, preferably at least two CpG motifs.

8. The composition or kit of anyone of the preceding claims, wherein the IL-7 antagonist is an interleukin-7 receptor (IL7R), binds to an IL7R or binds to IL-7, wherein preferably the IL-7 antagonist binding to IL7R binds to an alpha chain of IL7R (CD127).

9. The composition or kit of anyone of the preceding claims, wherein the IL-7 antagonist binding to IL7R or IL-7 is an antibody, or an antigen binding fragment thereof,
preferably the IL-7 antagonist binding to IL7R or IL-7 is a monoclonal antibody, or an antigen binding fragment thereof.

10. The composition or kit of anyone of the preceding claims, wherein the IL-7 antagonist is an antibody, preferably a monoclonal antibody, binding to IL7R (IL7R antibody), or an antigen binding fragment thereof, wherein the IL7R antibody, or the antigen binding fragment thereof, binds to the IL7R alpha chain.

11. The composition or kit of anyone of the preceding claims, further comprising a kinase inhibitor, preferably the kinase inhibitor is a tyrosine kinase inhibitor, more preferably the kinase inhibitor is a Bcr-Abl tyrosine-kinase inhibitor.

12. The composition or kit of any one of the preceding claims for use in treating or preventing lymphoblastic leukemia,
preferably the lymphoblastic leukemia is acute lymphoblastic leukemia or chronic lymphatic leukemia, more preferably acute lymphoblastic leukemia (ALL), again more preferably the ALL is B-cell precursor ALL (BCP-ALL).

13. The composition or kit for use according to claim 12, wherein the ALL or BCP-ALL is Ph-negative, Ph-positive or Ph-like, preferably Ph-positive or Ph-like, more preferably Ph-positive.

14. The composition or kit for use according to claim 12 or 13, wherein the ALL is BCR::ABL1-like ALL or **characterized by** the presence of a fusion gene encoding Bcr-Abl (Bcr-Abl positive, Bcr-Abl+) or a Philadelphia chromosome (Philadelphia chromosome-positive, Ph+), preferably the ALL is Bcr-Abl+ or Ph+.

15. The composition for use according to any one of claims 12-14, wherein the patient is resistant to treatment with kinase inhibitors, preferably tyrosine kinase inhibitors, more preferably Bcr-Abl tyrosine-kinase inhibitors.
